(19) Europäisches Patentamt
European Patent Office
Office européen des brevets

(11) **EP 2 285 815 B1**

(12) **EUROPÄISCHE PATENTSCHRIFT**

(45) Veröffentlichungstag und Bekanntmachung des Hinweises auf die Patenterteilung:
**12.10.2016 Patentblatt 2016/41**

(21) Anmeldenummer: **09757159.0**

(22) Anmeldetag: **12.05.2009**

(51) Int Cl.:
***C07H 1/08*** *(2006.01)*   ***C12N 15/10*** *(2006.01)*

(86) Internationale Anmeldenummer:
**PCT/EP2009/003363**

(87) Internationale Veröffentlichungsnummer:
**WO 2009/146775 (10.12.2009 Gazette 2009/50)**

(54) **VERFAHREN ZUR ISOLIERUNG VON KURZKETTIGEN NUKLEINSÄUREN**

METHOD FOR ISOLATING SHORT CHAIN NUCLEIC ACIDS

PROCÉDÉ D'ISOLATION D'ACIDES NUCLÉIQUES À CHAÎNES COURTES

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **30.05.2008 EP 08009941**

(43) Veröffentlichungstag der Anmeldung:
**23.02.2011 Patentblatt 2011/08**

(73) Patentinhaber: **Qiagen GmbH**
**40724 Hilden (DE)**

(72) Erfinder:
• **RITT, Christoph**
**40764 Langenfeld (DE)**
• **HORLITZ, Martin**
**40474 Düsseldorf (DE)**
• **SPRENGER-HAUSSELS, Markus**
**40822 Mettmann (DE)**

(74) Vertreter: **Roth, Carla**
**König-Szynka-Tilmann-von Renesse**
**Patentanwälte Partnerschaft mbB**
**Postfach 11 09 46**
**40509 Düsseldorf (DE)**

(56) Entgegenhaltungen:
**EP-A- 1 690 938        WO-A-99/61603**
**US-A1- 2007 106 071**

**Beschreibung**

Die vorliegende Erfindung betrifft ein Verfahren zur Isolierung und/oder Aufreinigung von Nukleinsäuren, insbesondere von kurzkettigen Nukleinsäuren.

Die Isolierung von Nukleinsäuren wie DNA und RNA aus pflanzlichen, tierischen oder menschlichen Materialien sowie aus Zellkulturen oder Viruskulturen erfolgt In der Regel nach einem einheitlichen Grundmuster: die Nukleinsäuren enthaltenen Ausgangsmaterialien werden zunächst - teilweise unter Verwendung von proteinabbauenden Enzymen - aufgeschlossen. In nachfolgenden Schritten können die einzelnen Bestandteile unter Verwendung verschiedenster Methoden abgetrennt werden. Darüber hinaus können Nukleinsäuren aus Prabenmaterialien Isoliert werden, in denen sie frei, d.h. nicht in Zellen, vorliegen. So können freie Nukleinsäuren in künstlichen Probenmischungen vorkommen, jedoch aber auch in natürlichen Proben wie bspw. Blut. Solche frei zirkulierenden Nukleinsäuren werden auch extrazelluläre Nukleinsäuren genannt.

Die meisten der aus dem Stand der Technik bekannten Verfahren zur Aufreinigung der Nukleinsäure basieren auf einem der beiden folgenden Abtrennungsprinzipien:

Die klassischen Verfahren beruhen auf einem einstufigen Prozess, in dem nach Zusatz eines Puffers, der in den meisten Fällen ein Chaotrop enthält, und nach Zusatz eines organischen Extraktionsmittels - meistens Phenol und/oder Chloroform - eine Extraktion durchgeführt wird. Die unerwünschten Begleitstoffe werden mit der organischen Phase verworfen. Die in der wässrlgen Phase verbleibenden Nukleinsäuren können anschießend durch eine Phasenseparation abgetrennt und damit isoliert werden. Der wesentliche Nachteil dieser Verfahrensweise besteht darin, dass neben der Verwendung giftiger und gesundheitsschädigender Stoffe wie Phenol und/oder Chloroform wasserlösliche Stoffe als Verunreinigung in der wässrigen Nukleinsäurelösung verbleiben, die in weiteren, sehr zeitaufwendigen Reinigungsschritten abgetrennt werden müssen.

Angesichts dieser Nachteile hat sich daher im Stand der Technik ein alternativer Prozess durchgesetzt, welcher auf der selektiven Adsorption von Nukleinsäuren an festen Trägermateriallen, wie beispielsweise Siliziumdioxid, basiert. Das nukleinsäurehaltige Ausgangsmaterial wird bei Bedarf lysiert, unter definierten Bedingungen mit dem Tragermaterial in Kontakt gebracht, so dass die Nukleinsäuren an das Trägermaterial binden können; ggf. werden Waschschritte durchgeführt. Optional wird im Anschluss die an den Träger gebundene Nukleinsäure von dem Trägermaterial mittels eines geeigneten Puffers eluiert.

Ein für eine Vielzahl unterschiedlicher Anwendungen geeignetes Verfahren zur Isolierung von Nukleinsäuren Ist beispielsweise in der US 5,234,809 (Boom) offenbart. Dort Ist ein Verfahren zur Isolierung von Nukleinsäuren aus nukleinsäurehaltigen Ausgangsmaterialien durch die Inkubation des Ausgangsmaterials mit einem chaotropen Puffer und einer DNA bindenden festen Phase beschrieben. Die chaotropen Puffer realisieren, sofern notwendig, sowohl die Lyse des Ausgangsmaterials wie auch die Bindung der Nukleinsäuren an die feste Phase. Das Verfahren ist gut geeignet, um Nukleinsäuren aus kleineren Probemengen zu isolieren. Ein auf einem ähnlichen Prinzip beruhendes Verfahren ist auch in WO93/11221 beschrieben.

EP 1 690 938 offenbart ein allgemeines Verfahren zur Aufreinigung von Nukleinsäuren unter Einsatz einer chaotropen Verbindung und/oder eines verzweigten Alkohols. Der Alkohol kann in einer Konzentration von 1% bis 100% vorliegen. EP 1 690 938 zielt nicht darauf ab, kurzkettige Nukleinsäuren mit einer Länge ≤ 1000 Nukleotiden zu isolieren. Spezifisch erwähnt ist die Isolierung von deutlich längeren Nukleinsäuren, Gelehrt wird, dass die Bindung von Nukleinsäuren allgemein verbessert werden kann, wenn die Bindung bei einer erhöhten Temperatur erfolgt.

WO 99/61603 lehrt ein Plasmid-Aufreinlgungsverfahren, bei dem chaotrope Salze zum Einsatz kommen, um die Probe zu lysieren und die Plasmide selektiv an ein Silikamaterial zu binden.

US 2007/106071 offenbart ein Verfahren zur Isolierung von Nukleinsäuren unterschiedlicher Länge, bei dem ebenfalls chaotrope Verbindungen eingesetzt werden. Das wesentliche Merkmal der offenbarten technischen Lehre liegt im Einsatz zweier verschiedener nukleinsäurebindenden festen Phasen, die eine unterschiedliche Porengröße und damit eine unterschiedliche Neigung zur Bindung von unterschiedlich langen Nukleinsäuren aufweisen.

Im Stand der Technik sind viele Verfahren zur Nukleinsäureaufreinigung bekannt, die aus der Kombination einer festen Phase mit einem chaotropen Puffer bestehen. Da bei allen bekannten Verfahren langkettige Nukleinsäuren mindestens genauso gut, in den meisten Fällen jedoch wesentlich besser an die feste Phase binden als kurzkettige Nukleinsäuren (beispielsweise mit einer Länge von weniger als 1000 bp, 500 bp oder sogar 300 bp), sind die Verfahren aus dem Stand der Technik nicht geeignet, um kurzkettige Nukleinsäuren effizient aufzureinigen bzw. sogar relativ zu den langkettigen Nukleinsäuren anzureichem.

Die schlechte Eignung, kurzkettige Nukleinsäuren aufzureinigen, ist vermutlich darauf zurückzuführen, dass die kurzkettigen Nukleinsäuren schlechter an das Trägermaterial binden, als langkettige Nukleinsäuren (bspw. genomische DNA). Daher gehen kurzkettige Nukleinsäuren bei den meisten der gängigen Aufreinigungsprozessen zu einem großen Teil verloren und finden sich nicht oder unterrepräsentiert unter den aufgerelnigten Nukleinsäuren. Für bestimmte Anwendungen ist es Jedoch gerade erwünscht, kurzkettige Nukleinsäuren zu isolieren, oder diese gegenüber langkettigen Nukleinsäuren anzureichern.

Um kurzkettige Nukleinsäuren bevorzugt anzureichern bzw. kurzkettige von langkettigen Nukleinsäuren zu trennen, wurden bereits verschiedene Prinzipien im Stand der Technik angewendet. DE 10 2006 045 391 beschreibt beispielsweise ein Verfahren zur Abtrennung langer Nukleinsäuren von kurzen Nukleinsäuren, bei dem die Probe mehrfach über speziell ausgestaltete feste Phasen gegeben wird. Ein anderes Verfahren beruht auf dem Einsatz spezieller Bindepuffer, die keine chaotropen Salze enthalten, sondern Zitronensäuresalze, um so insbesondere kurzkettige Nukleinsäuren zu binden (WO 2007/065934).

Die Aufreinigung/Anreicherung von kurzkettigen Nukleinsäuren (RNA und DNA) ist für verschiedene Anwendungsbereiche von zentraler Bedeutung. Ein Bereich, bei dem kurzkettige Nukleinsäuren eine zentrale Rolle spielen, ist die pränatale Diagnostik. Neben der eigenen frei zirkulierenden DNA befindet sich im Blut schwangerer Frauen zusätzlich noch frei zirkulierende DNA des Fötus. Es wird angenommen, dass sich die frei im Blut schwangerer Frauen zirkulierende fötale DNA von der frei zirkulierenden DNA der Mutter in ihrer Größe unterscheidet. Während die maternale frei zirkulierende DNA oftmals eine durchschnittliche Länge von über 500 bp besitzt, Ist ein überwiegender Teil der fötalen frei zirkulierenden DNA deutlich kleiner und besitzt Im Schnitt eine Länge von unter 500 bp. Dieser Größenunterschied zwischen den fötalen und den maternalen Nukleinsäuren könnte ausgenutzt werden, um fötale DNA anzureichern und damit besser untersuchen zu können, sofern geeignete Aufreinigungsmethoden zur Verfügung stünden. Die Nutzung der frei zirkulierende DNA fötalen Ursprungs zur pränatalen Diagnostik hätte den Vorteil, dass es gegenüber klassischen Methoden wie die Amniozentese oder der Chorionzottenbiopsie für den Fötus ungefährlich wäre und daher weniger Risiken bergen würde. Jedoch sind die Mengen an frei zirkulierender DNA fötalen Ursprungs im Blut sehr gering. Je nach Stadium der Schwangerschaft befinden sich in 1 ml Blut zwischen 20 und 260 Kopien fötaler DNA. Die Konzentration an frei zirkulierender fötaler DNA ist damit zwar gering, aber immer noch höher als die Konzentration an frei zirkullerenden fötalen Zellen. Ferner besteht das Problem, dass die Konzentration der frei zirkulierenden fötalen DNA im Vergleich zur frei zirkulierenden maternalen DNA äußerst gering ist, so dass bei der Gesamtisolation frei zirkulierender DNA aus maternalem Blut nur ein Bruchteil der Erbsubstanz vom Fötus stammt; der überwiegende Teil der Isolierten Erbsubstanz stammt von der Mutter. Durch den hohen Hintergrund an maternaler DNA ist in vielen Fällen die Detektion von Genabschnitten des Fötus erschwert, In manchen Fällen reicht die Sensitivität selbst so empfindlicher Nachweismethoden wie der Real-Time PCR nicht aus, um die fötale DNA nachweisen zu können.

Die bislang eingesetzten Methoden zur Isolation von frei zirkulierender DNA aus maternalem Blut reinigen oftmals im gleichen Maße die fötale wie die maternale DNA auf, so dass das ungünstige Verhältnis von fötaler zu maternaler DNA bestehen bleibt - die fötale DNA macht nur einen Bruchteil der Gesamt-DNA aus. Eine Anreicherung von fötaler DNA gegenüber der maternalen DNA erfolgt bislang nicht. Ferner werden die fötalen Nukleinsäuren sogar regelmäßig schlechter aufgereinigt, da kurzkettige Nukleinsäuren oftmals weniger gut bei der Aufreinigung erfasst werden. Eine effiziente Aufreinigung oder sogar spezifische Anreicherung der kurzkettigen fötalen DNA wäre jedoch vorteilhaft, weil damit die Sensitivität und damit die Verlässlichkeit einer pränatalen Diagnostik, die auf frei zirkulierender fötaler DNA basiert, wesentlich erhöht würde.

Der vorliegenden Erfindung liegt daher die Aufgabe zugrunde, ein Verfahren zur Isolierung und/oder Aufreinigung von Nukleinsäuren zur Verfügung zu stellen, mit dem sich auch kurzkettige Nukleinsäuren effizient isolieren/aufreinigen lassen. Ferner ist es Aufgabe der Erfindung, ein Verfahren zur Isolation von fötaler DNA aus maternalem Blut bereit zu stellen, das eine effektive Isolierung und/oder Anreicherung der fötalen DNA erlaubt.

Die Aufgabe wird vorliegend gelöst durch ein Verfahren zur Anreicherung von kurzkettigen Nukleinsäuren, insbesondere DNA und/oder RNA, mit einer Länge von < 1000bp, aus einem nukleinsäurehaltigen Ausgangsmaterial mit folgenden Verfahrensschritten:

(x) Bindung von langkettigen Nukleinsäuren an ein nukleinsäureblndendes Trägarmaterial, indem man das Ausgangsmaterial in Gegenwart wenigstens einer chaotropen Verbindung und wenigstens eines verzweigten und/oder unverzweigten Alkohols mit dem nukleinsäurebindenden Trägermaterial In Kontakt bringt, wobei der Alkohol in einer Konzentration $\leq$ 25% (v/v) vorliegt;

(a) Bindung der Im Durchbruch/Überstande aus Schritt (x) enthaltenden kurzkettigen Nukleinsäuren an ein nukleinsäurabindendes Trägermaterial, indem man den Durchbruch/Überstand in Gegenwart wenigstens einer chaotropen Verbindung und wenigstens eines verzweigten und/oder unverzweigten Alkohols mit dem nukleinsäurebindenden Trägermaterial in Kontakt bringt, wobei der Alkohol in einer Konzentration von 25 bis 40% (v/v) vorliegt;

(b) optional Elution der gebundenen kurzkettlgen Nukleinsäuren von dem nukleinsäurebindenden Trägermaterial.

Nach dem erfindungsgemäßen Verfahren wird die Immobilisierung der Nukleinsäuren an das Trägermaterial unter spezifischen Reaktionsbedingungen vorgenommen. Entscheidend für die effektive Bindung der kurzkettigen Nukleinsäuren an das Trägermaterial sind zwei Kornponenten, nämlich eine chaotrope Verbindung sowie ein verzweigter und/oder unverzweigter Alkohol. Besonders wichtig ist erfindungsgemäß die richtige Wahl der Alkoholkonzentration in Schritt a). Es hat sich gezeigt, dass bei der Wahl der richtigen Alkoholkonzentration kurzkettige Nukleinsäuren sehr gut oder sogar bevorzugt (gegenüber längerkettigen Nukleinsäuren) an das Trägermaterial binden. Eine niedrigere Alkoholkonzentration kann In gewissem Umfang durch eine Erhöhung der Konzentration an chaotropen Substanzen ausgeglichen werden. Insgesamt lassen sich bei Alkoholkonzentrationen am oberen Ende des definierten Bereichs jedoch bessere Resultate erzielen. Die Konzentration des Alkohols bewirkt eine Selektivität im Hinblick auf die Größenfraktionierung der gebundenen Nukleinsäuren. Bei höheren Alkoholkonzentrationen werden insbesondere kurzkettige Nukleinsäuren effizienter gebunden.

Die Alkoholkonzentration liegt in einem Bereich von 25 bis 40% (v/v), da in diesem Bereich kurzkettige Nukleinsäuren und insbesondere DNA besonders gut gebunden und damit Isoliert werden können. Je nach Feineinstellung der Bindungsbedingungen kann so auch eine Anreicherung der kurzkettigen Nukleinsäuren gegenüber den langkettigen Nukleinsäuren erfolgen. Dieser vorteilhafte Anreicherungseffekt wird insbesondere bei einer Alkoholkonzentration zwischen 25 und 35% (v/v), 25 bis 32% (v/v) und 28 bis 32% (v/v) erzielt. Bei diesen Bedingungen binden die kurzkettigen Nukleinsäuren besser und damit bevorzugt an das Trägermaterial als die langkettigen Nukleinsäuren wie auch die Beispiele zeigen. Auch die Konzentration an chaotropen Verbindungen beeinflusst die Bindung der Nukleinsäuren. Geeignete chaotrope Verbindungen und Konzentrationen werden nachfolgend beschrieben. Bei Wahl einer höheren Konzentration an chaotropen Verbindungen können auch geringere Alkoholkonzentrationen eingesetzt werden.

Optional können Waschschritte durchgeführt werden. Die an das Trägermaterial gebundenen Nukleinsäuren werden dann vom Trägermaterial abgetrennt, bspw. in an sich bekannter Weise eluiert, sofern eine Gewinnung der kurzkettigen Nukleinsäuren gewünscht ist. Die erfindungsgemäß isolierten/angereicherten Nukleinsäuren können dann in bekannter Weise weiterverarbeitet, also bspw. analysiert werden. Je nach geplanter anschließender Weiterverarbeitung oder Analyse ist es jedoch ebenfalls möglich, die Nukleinsäuren gebunden am Trägermaterial und damit ohne Elution einzusetzen. Ferner kann das Verfahren dazu eingesetzt werden, Nukleinsäuren aus einer Probe zu entfernen.

Für bestimmte Anwendungen ist es vorteilhaft, eine möglichst effiziente Anreicherung der kurzkettigen Nukleinsäuren gegenüber den langkettigen Nukleinsäuren zu erzielen; die kurzkettigen Nukleinsäuren sollen ohne bzw. mit möglichst wenig Hintergrund an langkettigen Nukleinsäuren gewonnen werden. In diesem Fall ist es wünschenswert, so wenig langkettige Nukleinsäuren wie möglich mit aufzureinigen. Um dies besonders effektiv zu erreichen, wird den eigentlichen Isolationsschritten a) und optional b) zur Abtrennung/Elution der kurzkettigen Nukleinsäuren ein Schritt (x) vorgelagert, durch den eine effiziente Abreicherung der langkettigen Nukleinsäuren erzielt wird. Durch die vorherige Abreicherung der langkettigen Nukleinsäuren können kurzkettige Nukleinsäuren in besonders reiner Form gewonnen werden.

Das Verfahren zur Anreicherung von kurzkettigen Nukleinsäuren, insbesondere DNA und oder RNA, mit einer Länge von <1000bp, aus einem nukleinsäurehaltigen Ausgangsmaterial weist erfindungsgemäß folgende Verfahrensschritte auf:

(x) Bindung von langkettigen Nukleinsäuren an ein nukleinsäurebindendes Trägermaterial, Indem man das Ausgangsmaterial in Gegenwart wenigstens einer chaotropen Verbindung und wenigstens eines verzweigten und/oder unverzweigten Alkohols mit dem nukleinsäurebindenden Trägermaterial in Kontakt bringt, wobei der Alkohol in einer Konzentration $\leq 25\%$ (v/v) vorliegt;

(a) Bindung der im Durchbruch/Überstand aus Schritt (x) enthaltenen kurzkettigen Nukleinsäuren an ein nukleinsäurebindendes Trägermaterial, indem man den Durchbruch/Überstand aus Schritt (x) in Gegenwart wenigstens einer chaotropen Verbindung und wenigstens eines verzweigten und/oder unverzweigten Alkohols mit dem nukleinsäurebindenden Trägermaterial in Kontakt bringt, wobei der Alkohol in einer Konzentration von 25 bis $\leq 40\%$ (v/v), vorzugsweise $\leq 35\%$ (v/v) und weiter bevorzugt $\leq 32\%$ (v/v) vorliegt;

(b) optional Elution der gebundenen kurzkettigen Nukleinsäuren von dem nukleinsäurebindenden Trägermaterial.

Gemäß dem erfindungsgemäßen Verfahrens ist den eigentlichen Isolationsschritten a) und b) zur Isolation/Aufreinigung der kurzkettigen Nukleinsäuren der Schritt (x) vorgelagert, durch den eine effiziente Abreicherung der langkettigen Nukleinsäuren erzielt wird. Im Bindungsschritt (x) erfolgt die Bindung der Nukleinsäuren an das Trägermaterial in Gegenwart von chaotropen Verbindungen und wenigstens einem verzweigten und/oder unverzweigten Alkohol. Entscheidend für die Abreicherung der langkettigen Nukleinsäuren sind wiederum die Bindungsbedingungen und insbesondere die Konzentration des während der Bindung in der Gesamtprobe vorhandenen Alkohols. Die Alkoholkonzentration ist in Schritt (x) $\leq 25\%$ (v/v). Es hat sich überraschenderweise gezeigt, dass eine Alkoholkonzentration um die 25% (v/v) eine Umkehr der Größenselektivität mit sich bringen kann. Unterhalb von 25 % (v/v), vorzugsweise unterhalb von 20 % (v/v), werden bevorzugt lang- bzw.

längerkettige Nukleinsäuren an das Trägermaterial gebunden, sofern die Konzentration an chaotropen Verbindungen entsprechend gewählt wird (siehe unten). Die kurzkettigen Nukleinsäuren binden bei diesen Bedingungen nicht bzw. schlechter an das Trägermaterial und befinden sich daher im Durchbruch/Überstand (In Abhängigkeit des eingesetzten Trägermaterials). Aus dem insbesondere die kurzkettigen Nukleinsäuren enthaltenden Durchbruch/Überstand aus Schritt (x) werden dann in den sich anschließenden Schritten a) und optional b) die kurzkettigen Nukleinsäuren isoliert und damit angereichert.

Durch den vorgelagerten Schritt (x) erfolgt letztlich zusätzlich eine Abreicherung der langkettigen Nukleinsäuren, so dass sich diese zumindest in geringerer Menge in dem Durch-bruch/Überstand befinden. Sofern gewünscht, können im Falle des 2-Schrittverfahrens die langkettigen Nukleinsäuren ebenfalls von dem in Schritt (x) gebundenen Trägermaterial eluiert und für andere Zwecke weiterverwendet werden.

Verschiedene Materialien und insbesondere biologische Materialen können als nukleinsäurehaltige Ausgangsmaterialien zum Einsatz kommen. Hierzu gehören bspw. Viren, Phagen und Zellen, wie z. B. Bakterien aber auch humane, tierische oder pflanzliche Zellen. Daneben eignet sich das Verfahren jedoch insbesondere auch zur Isolierung/Aufreinigung von freien Nukleinsäuren aus nicht-zellhaltigen Proben, oder entsprechend aufbereiteten Proben. Insbesondere eignet sich das erfindungsgemäße Verfahren bspw, für die Isolierung von Nukleinsäuren wie DNA und/oder RNA aus Probenmaterialen humanen oder tierischen Ursprungs, insbesondere klinischen Proben, wie Blut, Plasma, Serum, Mundspülflüssigkeit, Urin, Zerebralflüssigkeit, Sputum, Stuhl, Punktate, Epithelabstriche, Biopsien und anderen Geweben oder Kriochenmarkproben. Insbesondere ist das erfindungsgemäße Verfahren zur Isolierung von fötaler DNA aus maternalen Blutproben und Insbesondere Blutplasma geeignet. Ebenso Ist das erfindungsgemäße Verfahren insbesondere für die Isolierung frei zirkulierender Nukleinsäuren wie beispielsweise Tumor-DNA und -RNA aus Körperflüssigkeiten, wie insbesondere Plasma und/oder Serum geeignet.

In bestimmten Fällen kann die Probe ohne Vorbehandlung in dem erfindungsgemäßen Verfahren eingesetzt werden. In vielen Fällen muss die Probe jedoch zunächst durch eine geeignete Methode aufgeschlossen und das in der Probe enthaltene biologische Material freigesetzt werden. Verfahren zum Aufschluss von Proben und Zellen sind dem Fachmann bekannt und können chemischer, enzymatischer oder physikalischer Natur sein. Auch eine Kombination dieser Verfahren Ist möglich.

Hierbei können sich verschiedene Faktoren für unterschiedliche biologische Materialien als vorteilhaft herausstellen; prinzipiell sind folgende Methoden gut geeignet: Lyse mit Hilfe von ionischen und nicht-ionogenen Detergenzien, wie z. B. SDS, LiDS oder Sarkosyl in geeigneten Puffern, der Einsatz von chaotropen Salzen, wie beispielsweise Guanidiniumhydrochlorid (GHCL), GGuanidinlumthiozyanat (GTC), Guanidiniumisothiozyanat (GITC), Natriumiodid, Nathumperchlorat u. a.; mechanisches Auselnanderreißen, wie z. B. mittels einer French Prass, Ultraschall, Mahlen mit Glaskugeln, Nickelkugeln, Aluminium oder in flüssigen Stickstoff; enzymatische Lyse, beispielsweise mit Lysozym, Proteinasen, Proteinase K oder Zellulasen oder mittels anderen kommerziell erhältlichen Enzymen für die Lyse; Lyse der Zellen mittels Bakteriophagen oder Virusinfektionen; Gefriertrocknen; osmotischer Schock; Mikrowellenbehandlung; Temperaturbehandlung; beispielsweise Erwärmen oder Kochen oder Gefrieren, z. B. in Trockeneis oder flüssigem Stickstoff und Auftauen; alkalische Lyse.

Wie ausgeführt, stellen die vorstehenden Verfahren Stand der Technik zur Lyse da, die hinlänglich bekannt sind und daher nicht im Detail erläutert werden müssen.

Bei der Aufreinigung von frei zirkulierender Nukleinsäuren wie bspw. DNA aus einer Blutprobe, wie bspw. zur Aufreinigung fötaler DNA aus einer maternalen Probe, werden zunächst die Zellen und weitere feste Bestandteile des Blutes abgetrennt (bspw. durch Zentrifugation), und das so gewonnene Plasma weiterverarbeitet. Das Plasma ist üblicherweise frei von Zeilen und enthält die frei zirkulierende Nukleinsäure, bspw. maternale und fötale DNA. Bei der Aufreinigung von freien Nukleinsäuren aus Plasma ist keine eigentliche Zelllyse erforderlich, um die Nukleinsäuren freizusetzen, da diese schon in frei zirkulierender Form vorliegen. Dies gilt auch für andere Proben, bei denen die Nukleinsäuren frei vorliegen und sich entsprechend nicht in Zellen befinden. Frei zirkulierende Nukleinsäuren können jedoch komplexiert mit Proteinen und/oder anderen Stoffen vorliegen. Aus diesem Grunde wird das nukleinsäurehaltige Ausgangsmaterial, beispielsweise das Blutplasma, zunächst mit einem Release-Puffer behandelt, der sicherstellt, dass die Nukleinsäuren aus der komplexierten Form freigesetzt werden. Der Release-Puffer Ist In seiner Funktion und Zusammensetzung ähnlich einem Lyse-Puffer, der zum Zellaufschluss eingesetzt wird; durch den Release-Puffer werden in der Probe geeignete Bedingungen für die Freisetzung der Nukleinsäuren erzeugt, so dass diese nicht komplexiert vorliegen. Durch die Zugabe des Release-Puffers wird die Nukleinsäure der Aufreinigung besser zugänglich. Das erfindungsgemäße Verfahren kann entsprechend auch bei zellfrelen Ausgangsmaterialien eingesetzt werden. Der entsprechende Release-Puffer kann, je nach Zusammensetzung, auch als Lyse-Puffer fungieren, um Nukleinsäuren effektiv aufzureinigen bzw. anzureichern. In der Regel können die üblichen Lyse-Puffer auch als Release-Puffer eingesetzt werden.

Erfindungsgemäß ist der Einsatz von chaotrophaltigen Release - bzw. Lyse-Puffern besonders effektiv. Dies Insbesondere, da auch die Zusammensetzung des Release- bzw. Lyse-Puffers die Bedingungen beeinflusst, unter denen die Nukleinsäuren an das Trägermaterial binden. Die Bindungsbedingungen in der Probe, die erfindungsgemäß entscheidend für die Effektivität des erfindungsgemäßen Verfahrens sind, können daher auch durch geeignete Wahl des Release- bzw. Lyse-Puffers bspw. in Kombination mit einem Bindepuffer eingestellt werden. Release- bzw. Lysepuffer gemäß der vorliegenden Erfindung enthalten eine chaotrope Verbindung, wie z. B. GTC oder GHCL und ggf. ein Detergenz, wie z. B. SDS oder Tween. Diese Agenzien können in wässriger Lösung oder in einer Pufferlösung, d. h. als sog. Release- oder Lysepuffer vorliegen. Als Puffer kann jeder geeignete Puffer, wie beispielsweise Tris, Bicin, Tricin oder Phosphatpuffer eingesetzt werden. Alternativ kann das Lyse- bzw. Release Agens auch getrennt zugegeben werden. Geeignete Konzentrationen und Mengen der Lyse- bzw. Release Agenzien variieren in Abhängigkeit von den betreffenden Systemen, Art der Zellen etc. und können durch den Fachmann bestimmt werden. Für spezifische Anwendungen, insbesondere die Aufreinigung von fötaler DNA aus Blutproben haben sich beispielsweise Konzentrationen Im Bereich von 2 bis 7 M chaotrope Verbindungen, wie z, B. GTC, GHCL oder NaI oder Natriumperchlorat, 0,1 M bis 1 M alkalische Agenzien, wie z. B. NaOH sowie 0,1 bis 50 Gew% (w/v) Detergenzien, wie Insbesondere nicht-ionische Detergenzien wie beispielsweise Tween, bewährt.

Chaotrope Verbindungen sind auch während der Bindung der Nukleinsäuren an das Trägermaterial in dem Probergemisch vorhanden. Die chaotropen Verbindungen können bspw. aus dem Lyse- bzw. Release-Puffer stammen und/oder aber separat bspw. in Form eines Bindepuffers hinzugegeben werden. Entscheidend sind letztlich die Bindungsbedingungen in der Probe während der Bindung der Nukleinsäuren an das Trägermaterial. Hier kann die chaotrope Verbindung letztlich bis zur Löslichkeitsgrenze vorliegen. Der Einsatz chaotroper Verbindungen ist vorteilhaft for die effiziente Bindung der Nukleinsäure. Die Konzentration der chaotropen Verbindungen In der Probe liegt während der Bindung bevorzugt in einem Bereich von 1 bis 10 mol/l besonders bevorzugt bei 2 bis 6 mol/l. Geeignete Verbindungen sind beispielsweise Natriumiodid, Natriumperchlorat, Guadiniumthiocyanat, Guanidiumisothiocyanat und Guanidiumhydrochlorid.

Ferner kann die Probe während der Bindung Detergenzien, wie bspw. nicht-ionische Detergenzien und insbesondere Tween aufweisen, Die Detergenzien können entweder mit dem Release/Lysepuffer zugegeben werden, oder Bestandteil des Bindepuffers sein. Detergenzien bewirken eine effiziente Solubilisierung verschiedener Bestandteile in der Probe, so bspw. von Serum- und Plasmabestandteilen. Dadurch kann eine Verstopfung des nukleinsäurebindenden Trögermaterials verhindert werden, Dies ist Insbesondere bei Einsatz einer Silikamembran von Vorteil.

Wie ausgeführt, ist die Konzentration des Alkohols in der Probe während der Bindung der Nukleinsäuren an das Trägermaterial bei dem erfindungsgemäßen Verfahren entscheidend. Bevorzugt werden kurzkettige verzweigte oder unverzweigte Alkanole mit einem bis fünf Kohlenstoffatomen, wie z. B. Methanol, Ethanol, Propanol, Isopropanol, Butanole oder Pentanole eingesetzt. Auch Mischungen an entsprechenden Alkoholen können eingesetzt werden. Besonders bevorzugt wird Isopropanol oder ein Alkohol bzw. Alkoholgemisch mit Isopropanol ähnlichen Eigenschaften eingesetzt. Je nach Bindeschritt (Bindeschritt (x) zur Abreicherung der langkettigen Nukleinsäuren oder Bindeschritt a) zur Bindung der kurzkettigen Nukleinsäuren) variiert die Konzentration des Alkohols.

Weil im Vorschritt (x) die langkettigen Nukleinsäuren abgereicht werden, kommen Alkoholkonzentrationen zum Einsatz, die die Bindung der langkettigen Nukleinsäuren an das Trägermaterial fördern, um diese dadurch aus der Probe entfernen zu können. Der Durchbruch bzw. der Überstand enthält die gewünschten kurzkettigen Nukleinsäuren aufgrund der Abreicherung zu einem größeren Anteil.

Um eine Bindung der langkettigen Nukleinsäuren zu fördern und damit die Abreicherung zu verbessern, liegt die Konzentration des Alkohols erfindungsgemäß während der Bindung der Nukleinsäuren an das Trägermaterial bei $\leq$ 25% ist, besonders bevorzugt bei $\leq$ 20% (v/v). Bei diesen Bedingungen werden insbesondere langkettige Nukleinsäuren gut gebunden. Dies gilt insbesondere in Gegenwart von $\geq$1 mol/l an chaotropen Verbindungen, bevorzugt $\geq$2 mol/l, Insbesondere $\geq$2,4 mol/l. Vorzugsweise beträgt die Konzentration an Chaotrop zur Abreicherung der langkettigen Nukleinsäuren zudem $\leq$ 4 mol/l, weiter bevorzugt $\leq$ 3,5 mol/l, noch weiter bevorzugt $\leq$ 3,2 mol/l und am meisten bevorzugt $\leq$ 3,1 mol/l. Die bevorzugte Konzentration an chaotroper Verbindung in der Probe hängt zudem von der Art bzw. Stärke der chaotropen Verbindung ab. Besonders starke chaotrope Verbindungen wie bspw. Guanidinumthiocyanat sollten in geringer Konzentration eingesetzt werden, um die Bindung langkettiger Nukleinsäuren zu fördern. Vorzugsweise wird Guanidinumhydrochlorid in einer Konzentration von $\geq$2,5 mol/l und $\leq$ 3,1 mol/l bei einer Alkoholkonzentration zwischen 15 und 25%, vorzugsweise bei ca. 20% eingesetzt, um die längerkettigen Nukleinsäuren zu binden und damit abreichern zu können.

Im Schritt a) des 2-Schritt Verfahrens wird eine höhere Alkoholkonzentration zur bevorzugten Bindung der kurzkettigen Nukleinsäuren eingesetzt. Dies vorzugsweise bei einer in etwa gleich bleibenden Konzentration an chaotroper Verbindung. Zur besseren Bindung der kurzkettigen Nukleinsäuren ist die Alkoholkonzentration in der Probe während der Bindung In einem Bereich von 25 bis 40% (v/v), bevorzugt 25 bis 36%. Bei Konzentrationen oberhalb von ca. 25% (v/v) wurde sogar festgestellt, dass kurzkettige Nukleinsäuren gegenüber langkettigen Nukleinsäuren angereichert werden konnten, insbesondere bei vorheriger Abreicherung der Nukleinsäuren. Dies gilt insbesondere bei Einstellung der Konzentration an chaotropen Verbindungen; geeignete Konzentrationen sind oben beschrieben. Dieses Ergebnis ist insbesondere für die Aufreinigung extrazellulärer Nukleinsäuren wie bspw. fötaler DNA aus Blutproben vorteilhaft, da die Proben wie bspw. maternales Blutplasma normelerweise überwiegend langkettige Nukleinsäuren enthalten. Mit dem erfindungsgemäßen Verfahren kann daher sogar eine effektivere Anreicherung der kurzkettigen Nukleinsäuren erzielt werden. Besonders bevorzugt enthält das Probengemisch während der Bindung der Nukleinsäuren an das Tragermaterial zur bevorzugten Bindung der kurzkettigen Nukleinsäuren ca. 30% (v/v) an Alkohol. Dadurch wird ein effektives Verfahren zur Anreicherung von kurzkettigen Nukleinsäuren zur Verfügung gestellt, das eine >2-fache, > 5-fache oder sogar mehr als 10-fache Anreicherung der kurzkettigen Nukleinsäuren erzielt. Allgemein kann das Anreicherungspotential des Verfahrens durch Vergleich der Ausbeuten eines 200 bp langen Nukfeinsäurefragmentes und eines 1000 bp langen Nukleinsäurefragmentes bestimmt werden. Damit ist das erfindungsgemäße Verfahren den Verfahren des Standes der Technik auch deutlich überlegen.

Gemäß einer Ausführungsform des 2-Schrittverfahrens liegt in Schritt (a) der Alkohol in der Probenmischung in einer Konzentration vor, die ausgewählt ist aus der Gruppe

- $\geq 25 \text{ bis} \leq 36\% \text{ (v/v)}$;

- $\geq 25 \text{ bis} \leq 35\% \text{ (v/v)}$;

- $\geq 25 \text{ bis} \leq 32\% \text{ (v/v)}$;

- $\geq 28 \text{ bis} \leq 32\% \text{ (v/v)}$.

Beim 2-Schrittverfahren, bei dem in Schritt (x) eine Abreicherung der langkettigen Nukleinsäuren erfolgt, ist die Konzentration der chaotropen Verbindungen in Schritt a) $\geq$ der Konzentration an chaotropen Verbindungen in Schritt (x). Es werden deutlich bessere Ergebnisse erzielt, wenn die Konzentration der chaotropen Verbindungen beim Übergang des Verfahrensschritt (x) zum dem Verfahrensschritt a) nicht gesenkt wird. Dies insbesondere, da die Alkoholkonzentration in Schritt (a) erhöht wird. Bevorzugt wird im eigentlichen Isolationsschritt a) sogar eine höhere Konzentration an chaotropen Verbindungen eingesetzt als in Schritt (x), um die Bindung der kurzkettigen Nukleinsäuren an das Trägermaterial zu fördern.

Gemäß einer Ausführungsform weist das erfindungsgemäße Verfahren wenigstens eines der folgenden Merkmale auf:

- dass die Konzentration der chaotropen Verbindungen in der Mischung in Schritt (x) und/oder Schritt (a) $\geq 1$ mol/l bis zur Löslichkeitsgrenze ist; und/oder
- dass die Konzentration der chaotropen Verbindungen in der Mischung in Schritt (x) und/oder Schritt (a) $\geq 2$ mol/l, $\geq 2,4$ mol/l oder $\geq 2,6$ mol/l ist; und/oder
- dass die Konzentration der chaotropen Verbindungen in Schritt (a) $\geq$ der Konzentration der chaotropen Verbindungen In Schritt (x) ist; und/oder
- dass das nukleinsäurehaltige Ausgangsmaterial zuvor mit einem Lyse-Puffer behandelt wird; und/oder
- dass das nukleinsäurehaltige Ausgangsmaterial keine Zellen enthält und keine Zelllyse durchgeführt wird; und/oder
- dass das nukleinsäurehaltige Ausgangsmaterial zuvor mit einem Release-Puffer behandelt wird; und/oder
- dass keine Phenol-Extraktion durchgeführt wird; und/oder
- dass die isolierten Nukleinsäuren mit DNAse behandelt werden; und/oder
- dass wenigstens 30% an kurzkettigen Nukleinsäuren mittels des Verfahrens isolierbar sind; und/oder
- dass wenigstens 50% an kurzkettigen Nukleinsäuren mittels des Verfahrens isolierbar sind; und/oder
- dass wenigstens 60% an kurzkettigen Nukleinsäuren mittels des Verfahrens isolierbar sind; und/oder
- dass eine wenigstens 2-fache Anreicherung der kurzkettigen Nukleinsäuren erzielt wird; und/oder
- dass eine wenigstens 5-fache Anreicherung der kurzkettigen Nukleinsäuren erzielt wird; und/oder
- dass eine wenigstens 10-fache Anreicherung der kurzkettigen Nukleinsäuren erzielt wird; und/oder
- dass kurzkettige Nukleinsäuren mit einer Länge isoliert und/oder angereichert werden, die ausgewählt sind aus der Gruppe von Nukleinsäuren mit einer Länge von $\leq$ 500bp, $\leq$ 400bp und/oder $\leq$ 300bp und/oder $\geq$ 50bp undloder $\geq$ 10bp; und/oder
- extrazelluläre Nukleinsäuren isoliert werden.

Das nukleinsäurebindende Trägermaterial ist vorzugsweise eine nukleinsäurebindende Festphase aus der Gruppe der silikahaltigen Materialien, Silikagel, Siliziumdioxid, Glas, Zeolith, Aluminiumoxid, Titandioxid, Zirkoniumdioxid, Kaolin, Kieseigel, Keramik oder polymere Trägermaterialien sowie Polystyrolkügelchen. Entscheidend ist letztlich, dass das Trägermaterial in der Lage ist, Nukleinsäuren zu binden. Besonders bevorzugt werden Silikamaterialien eingesetzt. Hier haben sich sowohl der Einsatz von Silikamembranen als auch von magnetischen Partikeln bewährt, die eine Silika- oder Glasoberfläche aufweisen. Diese können Im Wesentlichen perl- oder kugelförmig sein und weisen vorzugsweise eine Partikelgröße im Bereich von 0,02-30 $\mu$m, vorzugsweise 0,05-15 $\mu$m und besonders bevorzugt von 0,1-10 $\mu$m auf. Magnetische Silikapartikel, die vorteilhafter Weise im erfindungsgemäßen Verfahren eingesetzt werden können, sind z. B. in der internationalen Patentanmeldung WO 01/71732 beschrieben, worauf hiermit voll inhaltlich Bezug genommen wird.

Nach der Inkubation der Trägermatrix mit dem nukleinsäurehaltigen Material erfolgt die Abtrennung der Nukleinsäuren von der restlichen Probenflüssigkeit. Dies wird allgemein durch die Separation der erfindungsgemäß an die Partikel gebundenen Nukleinsäuren - bei Verwendung von magnetischen Silikapartikeln mit Hilfe eines Magnetfeldes - erreicht. Beispielsweise können die Magnetpartikel an die Wand des Gefäßes, in welchem die Inkubation stattgefunden hatte, gezogen werden. Daraufhin kann die Flüssigkeit mit den Inhaltsstoffen der Probe, die nicht an die magnetischen Partikel gebunden haben, entfernt werden. In Schritt (x) würde der Überstand weiterverarbeitet werden (die langkettigen Nukleinsäuren sind an die magnetischen Partikel gebunden); in Schritt a) werden die kurzkettigen Nukleinsäuren an die magnetischen Partikel gebunden und in Schritt b) ggf. nach Waschschritten optional von den Partikeln eluiert. Die Verarbeitung hängt von der Art des Gefäßes ab, in dem die Inkubation stattgefunden hat. Geeignete Verfahrensschritte zum Entfernen der Flüssigkeit sind z. B. abdekantieren, abpipettieren, abfiltrieren oder absaugen der Flüssigkeit.

Wie ausgeführt, können bspw. auch Silikamembranen eingesetzt werden. Flüssigkeiten können hier durch Zentrifugation oder Anlegung von Vakuum oder durch Druck entfernt werden. In vielen Anwendungen des erfindungsgemäßen Verfahrens, insbesondere bei der Aufrefnigung von fötaler DNA aus maternalen Blutproben, werden die aufzureinigenden Nukleinsäuren in geringen Konzentrationen In großen Volumen vorliegen. Bei Einsatz einer Silikamembran besteht im Stand der Technik oft das Problem, dass große Probenvolumina die Membran verstopfen können. Nach dem erfindungsgemäßen Verfahren tritt dieses Problem jedoch nicht auf, da spezielle Puffersubstanzen/-konzentrationen eingesetzt werden. Hier ist es insbesondere vorteilhaft, wenn Detergenzien eingesetzt werden.

Nukleinsäuren, die mit dem vorliegenden Verfahren isoliert werden können, sind bspw. DNA, RNA, mRNA, mitochondriale, epigenetisch modifizierte, einzelsträngige, doppelsträngige, zirkuläre, Plasmid, Cosmid, artifizielle oder synthetische Nukleinsäuren, sowie cDNA und Fragmente davon. Das erfindungsgemäße Verfahren eignet sich insbesondere zur Anreicherung von kurzkettigen Nukleinsäuren (bspw. DNA und RNA in jeglicher Form, einschließlich nicht kodierender RNA wie bspw. miRNA oder synthetische Nukleinsäuren) mit einer Länge von $\leq 1000bp$, $\leq 800bp$, $\leq 500\ bp$, oder $\leq 300\ bp$. Gemäß einer Ausführungsform werden kurzkettige Nukleinsäuren mit einer Länge isoliert und/oder angereichert, die ausgewählt sind aus der Gruppe von Nukleinsäuren mit einer Länge von $\leq 500bp$, $\leq 400bp$, $\leq 300bp$ und/oder $\geq 50bp$, $\geq 100bp$. Bevorzugt wird DNA und/oder RNA aufgereinigt. Besonders effizient lässt sich DNA oder RNA einer Länge $\geq 50$ Nukleotiden aufreinigen. Zur Gewinnung von kurzkettiger RNA wird die aufgereinigte Nukleinsäure vorzugsweise mit DNase behandelt.

Die Größe der Isolierten Nukleinsäuren kann auch durch Wahl der Alkoholkonzentration insbesondere in Kombination mit der Konzentration an chaotropen Agenzien variiert/gesteuert werden. Das erfindungsgemäße Verfahren ermöglicht eine effektive Isolierung auch von kurzkettigen Nukleinsäuren. Durch die geeignete Einstellung der Bindebedingungen wie hier beschrieben kann auch eine spezifische Anreicherung der kurzkettigen Nukleinsäuren erzielt werden. Das Verfahren eignet sich insbesondere zur Anreicherung von fötalen Nukleinsäuren, wie auch die nachfolgenden Beispiele belegen.

Das erfindungsgemäße Verfahren kann daher in vorteilhafter Weise eingesetzt werden, um extrazelluläre Nukleinsäuren wie bspw. fötale DNA aus maternalem Blut zu isolieren. Mit der vorliegenden Erfindung wird daher auch ein Verfahren zur Anreicherung von fötalen Nukleinsäuren aus einer Blutprobe, insbesondere aus Blutplasma oder -serum, zur Verfügung gestellt, das dadurch gekennzeichnet ist, dass das erfindungsgemäße Verfahren zur Isolation/Aufreinigung von kurzkettigen Nukleinsäuren durchgeführt wird. Einzelheiten des Verfahrens sind oben dargelegt.

Weitere Anwendungsbereiche finden sich jedoch beispielswelse in der Forensik und in anderen Bereichen, wo die Aufreinigung kleiner Nukleinsäuren entscheidend ist. Darüber hinaus kann das erfindungsgemäße Verfahren auch Im Rahmen der Diagnostik eingesetzt werden, um bspw. frei zirkulierende Tumor-Nukleinsäuren aus einer Probe, wie bspw. Blut aufzureinigen.

Mit der vorliegenden Erfindung wird ferner ein Verfahren zur Anreicherung von Nukleinsäuren aus einer Probe, außer zur Anreicherung von fötalen Nukleinsäuren aus einer Blutprobe, zur Verfügung gestellt, das dadurch gekennzeichnet ist, dass das erfindungsgemäße Verfahren zur Isolation/Aufreinigung von kurzkettigen Nukleinsäuren durchgeführt wird. Einzelheiten des Verfahrens sind oben dargelegt.

Das erfindungsgemäße Verfahren ist insbesondere für die Anwendung in der Diagnostik geeignet Es ist auch automatisierbar und daher zur Anwendung auf entsprechenden Aufreinigungsrobotern einsetzbar.

Ferner wird ein Kit zur Isolierung und/oder Aufreinigung von Nukleinsäuren, insbesondere von kurzkettigen Nukleinsäuren, aus einem nukleinsäurehaltigen Ausgangsmaterial offenbart, aufweisend Puffer und/oder Reagenzien und optional wenigstens ein nukleinsäurebindendes Trägermaterial für die Durchführung des erfindungsgemäßen Verfahrens. Einzelheiten des Verfahrens sind oben dargelegt.

Ein entsprechender Kit kann zur Aufreinigung von fötaler DNA aus einer Blutprobe eingesetzt werden. Darüber hinaus wird ein entsprechender Kit zur Aufreinigung von kurzkettigen Nukleinsäuren aus einer Probe, außer zur Anreicherung von fötaler DNA aus einer Blutprobe, zur Verfügung gestellt.

Die beschriebenen Kits sind insbesondere im Bereich der Diagnostik und für medizinische Anwendungen einsetzbar. Sie können automatisiert eingesetzt werden.

BEISPIELE

Die vorliegende Erfindung wird nunmehr anhand von Beispielen erläutert, soweit von den Ansprüchen erfasst. Die durchgeführten Versuche erfolgten anhand der nachfolgend beschriebenen Versuchsvorschriften.

Ausgangspunkt für die Anwendung des erfindungsgemäßen Verfahrens zur Isolation von frei zirkulierender fötaler DNA aus matemalem Blut mit gleichzeitiger Anreicherung der fötalen DNA relativ zur maternalen DNA ist der in der Literatur beschriebene Befund, dass sich die durchschnittliche Länge von beiden Nukleinsäurespezies unterscheidet. Während die durchschnittliche Länge fötaler DNA kürzer als 500 bp ist, Ist die durchschnittliche Länge maternaler DNA länger als 500 bp.

**Beispiel 1: Test zur Bestimmung der durchschnittlichen Länge fötaler und maternaler DNA**

Drei verschiedene Plasma-Pools, die aus Blut von mit männlichen Föten schwangeren Frauen gebildet waren, wurden für die Untersuchung der Größenverteilung der sich darin befindlichen frei zirkulierenden DNA verwendet. Die Plasma-Pools waren Pool A, B und C. Pool A beinhaltete Plasma aus Blutproben von schwangeren Frauen im ersten bis dritten Trimester der Schwangerschaft. Pools B und C beinhalteten jeweils Plasma aus Blut, das den schwangeren Frauen im ersten und zweiten Trimester der Schwangerschaft abgenommen wurde; ein Zeitpunkt, der noch geringere Mengen frei zirkulierender fötaler DNA enthält, aber für den klinischen Diagnosezeitpunkt von größerer Relevanz ist.

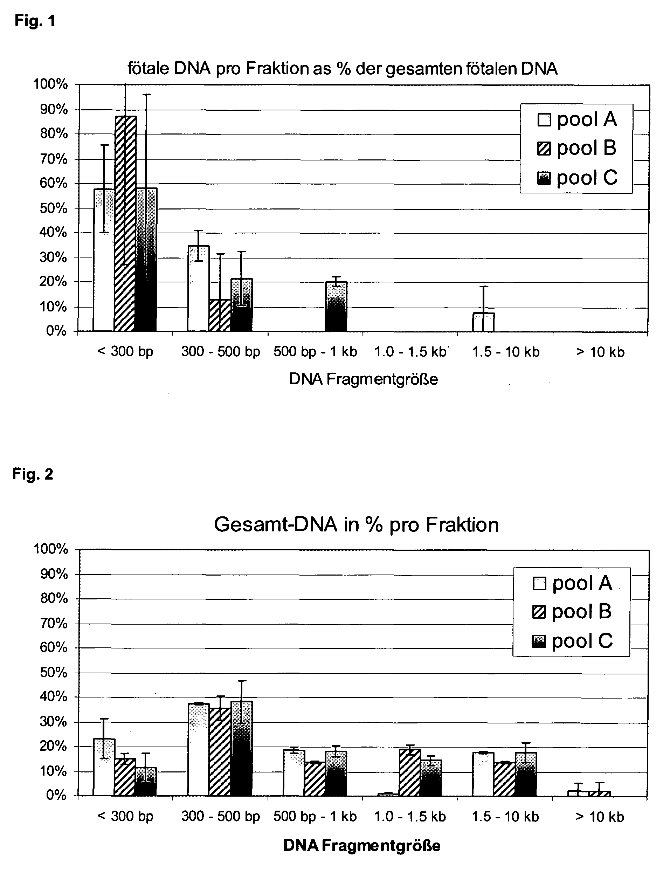

Als Ausgangsmaterial wurden 10ml Plasma und bei Pool A 5ml Plasma, verwendet. Vorgegangen wurde nach dem QIAamp Blood DNA Midi Protokoll (QIAGEN), welches auf ein Volumen von 10 ml angepasst wurde. Eluiert wurde jeweils mit 300 $\mu$l AE Puffer (QIAGEN, kommerziell erhältlich). Nach der Elution wurde eine Ethanol/Natriumacetat-Fällung durchgeführt und das getrocknete Pellet In 15 $\mu$l Puffer EB (QIAGEN, kommerziell erhältlich) aufgenommen. Nach einer Agarosegelelektrophorese wurden die einzelnen Größenfraktionen aus dem Gel herausgeschnitten und eine Gelextraktion nach dem QIAquick Vakuum-Protokoll zur Gelextraktion durchgeführt. Pro Gelfraktion wurde in 100 $\mu$l eluiert; in die nachfolgende PCR wurden jeweils 20 $\mu$l der Eluate in einer Doppelbestimmung eingesetzt. Amplifiziert wurde mit entsprechenden Primern zum einen der SRY Locus zum Nachweis der frei zirkulierenden fötalen DNA. SRY ist nur bei männlichen Individuen nachweisbar. Da ausschließlich Blut von Schwangeren verwendet wurde, die sicher einen männlichen Fötus austragen, waren alle SRY Signale auf DNA fötalen Ursprungs zurückzuführen. Amplifiziert wurde ferner mit entsprechenden Primern der c-myc Locus zum Nachweis der gesamten frei zirkulierenden DNA Im maternalen Blut. Die Amplifikation wurde auf einem ABI 7500 Instrument durchgeführt (Applied Biosystems). Dabei ergab sich das in Fig. 1 und Fig. 2 gezeigte Ergebnis.

Fig. 1 zeigt die Größenverteilung der fötalen DNA in Abhängigkeit der verwendeten Pools.
Fig. 2 zeigt die Größenverteilung der Gesamt-DNA in Abhängigkeit der verwendeten Pools.

Dieses Experiment zeigt, dass, wie in der Literatur beschrieben, die fötale DNA nur in kurzen Fragmenten vorliegt. Dabei liegt der Hauptteil deutlich in der Fraktion kleiner 300 bp, ein signifikanter Teil zeigt Fragmentlängen von 300 bis 500 bp. Nur sehr wenig der frei zirkulierenden fötalen DNA im maternalen Blut ist länger als 500 bp. Auf der anderen Seite ist nicht alle maternale DNA größer als 500 bp. In etwa die Hälfte der maternalen DNA, die frei im Blut zirkuliert, ist ebenfalls nur 500 bp lang und kürzer, die andere Hälfte ist jedoch deutlich länger als 500 bp. Mittels einer Größenfraktionierung der isolierten/angereicherten Nukleinsäuren lässt sich demnach eine signifikante, relative Anreicherung fötaler DNA gegenüber maternaler DNA erreichen.

**Beispiel 2**

Um die unterschiedliche Größenverteilung von fötaler DNA (der überwiegende Anteil ist kürzer als 300 bp, siehe Beispiel 1) und maternaler DNA (in der Hauptsache länger als 500 bp) zu simulieren, wurden in das Blutplasma als Hintergrund zwei unterschiedliche PCR-Amplifikate hinzugefügt. Ein 219 bp Fragment soll dabei die fötale DNA, ein Fragment von 1018 bp Länge die maternale DNA simulieren. In einem ersten Experiment wurden dazu relativ hohe Mengen dieser PCR-Amplifikate verwendet, nämlich jeweils 2x 10$^6$ Kopien in 600 $\mu$l Plasma. Vorgegangen wurde dabei nach folgendem Protokoll:

Zu 600 $\mu$l Plasma in einem 5 ml Gefäß wurden 90 $\mu$l Protease (QIAGEN) und 600 $\mu$l Puffer AL (QIAGEN, kommerziell erhältlich), der Guanidin enthält, gegeben. Nach Mischen durch Vortexen wurde der Ansatz zur Lyse für 15 min bei 56 "C inkubiert. Nach der Lyse wurden die PCR Amplifikate von 219 oder 1018 bp Länge zum Lysat hinzugegeben. Die Bindebedingungen wurden mit 100 $\mu$l Isopropanol so eingestellt, dass dadurch in der Gesamtprobe eine Konzentration von 6,9 % (w/v) Isopropanol resultierte.

Nach Mischen durch Vortexen wurde für 10 min bei Raumtemperatur inkubiert. Zur Bindung wurden 50 $\mu$l MagAttract magnetische Partikel mit Silikaoberfläche (QIAGEN) hinzugegeben und der Ansatz für 5 min auf einem Schüttler binden gelassen. Nach der Bindung wurden die Partikel mittels eines Magneten vom Überstand separiert und der Überstand abgenommen. Der Überstand wurde bis zur weiteren Behandlung bei 4 °C aufbewahrt.

### Behandlung der MagAttract Partikel

Nach Abnahme des Überstandes wurden die Magnetpartikel mit 750 $\mu$l Puffer AW1 (QIAGEN, kommerziell erhältlich) für 5 min auf einem Plattenschüttler gemischt und anschließend die Partikelsuspension in ein 1,5 ml Reaktionsgefäß überführt. Nach magnetischer Separation in diesem Gefäß wurde der Überstand abgenommen und verworfen. Anschließend wurden die Partikel noch (jeweils nach 5 min Inkubation auf einem Schottler) nacheinander mit 750 $\mu$l Puffer AW2 (QIAGEN, kommerziell erhältlich) und 750 $\mu$l Ethanol gewaschen. Nach dem Alkoholwaschschritt wurden die Partikel in einem Helzblock für 10 min bei 56 °C getrocknet. Zur Elution der an die Partikel gebundenert Nukleinsäuren wurden 200 $\mu$l RNase freies Wasser (QIAGEN) verwendet, wobei zur Elution erneut für 5 min geschüttelt wurde. Das Eluat wurde nach Magnetseparation In ein neues Gefäß überführt.

### Behandlung des Überstandes der Bindung

Der Überstand/Durchbruch der Bindung (das nicht an die Magnetpartikel gebundene Material) wurde wie folgt aufgearbeitet. Der Überstand nach der Bindung wurde mit 2 ml Puffer B6 (2,5 M GuHCl, 50% Isopropanol) versetzt, so dass eine Konzentration an Isopropanol von 32,4% (v/v) resultierte, durch Vortexen gemischt und 10 min bei Raumtemperatur inkubiert.

Nach der Inkubation wurden 50 $\mu$l MagAttract Partikel hinzugefügt und genau so vorgegangen, wie für den ersten Bindeansatz beschrieben (Waschen mit AW1 (QIAGEN, kommerziell erhältlich), AW2 (QIAGEN, kommerziell erhältlich), Ethanol, Elution in RNase freiem Wasser). Alternativ wurden zum Überstand nach der Bindung 2 ml 100% Isopropanol (Puffer B11) hinzugegeben, so dass eine Konzentrationen an Isopropanol von 61,9% (v/v) resultierte. Von den Eluaten wurden gleiche Aliquots abgenommen und mit Hilfe einer real-time PCR ein 119 bp Amplicon amplifiziert, das sowohl aus dem 219 bp Fragment als auch aus dem 1018 bp Fragment ein Identisches Fragment ergibt. Der Nachweis des Amplicons erfolgte während der PCR mit Hilfe von SYBR Green.

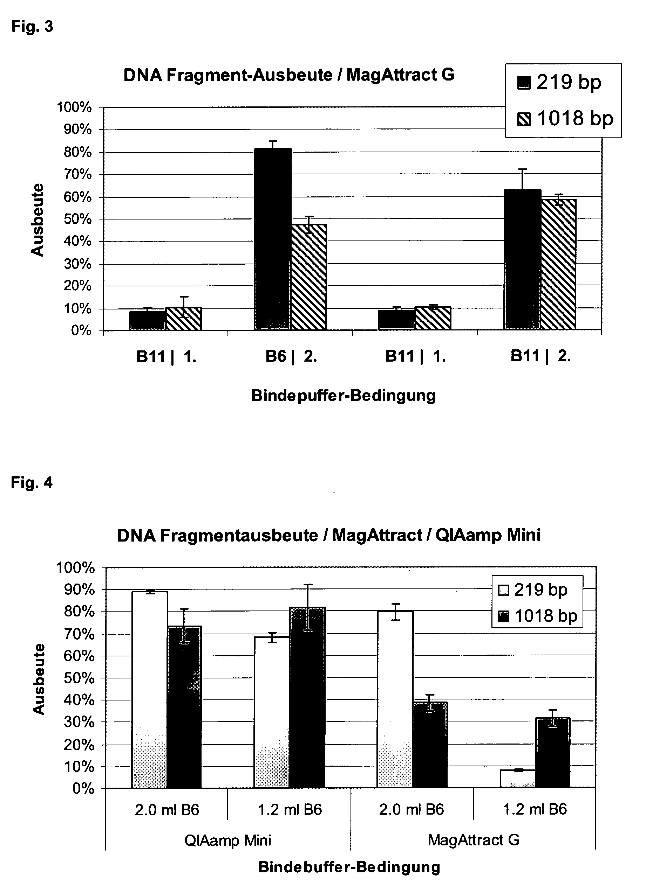

Dabei ergab sich das in Fig. 3 gezeigte Bild.

Dieses Experiment zeigt, dass sowohl vom 219 bp Fragment als auch vom 1018 bp Fragment unter den gewählten Bindebedingungen nur wenig Nukleinsäure an die ersten MagAttract Partikel bindet, jeweils etwa nur 10%. Bei neuer Einstellung der Bindebedingungen mit Puffer B6 bzw. B11 ergibt sich jedoch überraschenderweise ein Unterschied in der Ausbeute In Abhängigkeit der Fragmentgröße. Während mit Hilfe von Puffer B6 über 80% der kurzen DNA (die die fötale DNA repräsentiert) wiedergefunden werden können, ergibt sich für die längere DNA (die die maternale DNA repräsentiert) nur eine Ausbeute von etwa 50%. Beim Einsatz von Puffer B11 hingegen ergeben sich keine wesentlichen Unterschiede In der Ausbeute zwischen den beiden DNA-Fragmentlängen.

Dieser Versuch zeigt, dass mit Hilfe eines Zwei-Schritt-Bindesystems mit Hilfe von zwei festen Phasen unter geeigneten Bedingungen die fötale DNA relativ zur maternalen DNA angereichert werden kann, wobei nur sehr geringe Ausbeuteverluste an fötaler DNA zu verzeichnen sind.

### Beispiel 3

Vorgegangen wurde wie in Beispiel 2 beschrieben, nur dass diesmal nur 200.000 Kopien der definierten Fragmente eingesetzt wurden, um ein realistischeres Bild der tatsächlichen frei zirkulierenden Kopienzahlen im Blut zu simulieren. Gebunden an die Matrix unter der ersten Bedingung wurde diesmal mit 100 $\mu$l Puffer B11 (siehe oben) + 1,2 ml Puffer B6, so dass eine Konzentration an Isopropanol von 20,3% (v/v) resultierte, (siehe oben). Zur alternativen Bedingung für die Bindung an die Matrix wurden 100 $\mu$l Puffer B11 und 2,0 ml Puffer B6 zum Plasmalysat hinzugegeben. Zusätzlich wurden die DNA-Fragmente unter den beiden o.g. Pufferbedingungen im Bindeansatz jeweils an eine MagAttract Matrix bzw. QIAamp Mini Columns gebunden.

Dafür wurde wie folgt vorgegangen. Die Puffer B11 und B6 wurden zum Überstand gegeben, gemischt und für 10 min bel Raumtemperatur inkubiert. Das Lysat zweier Proben wurde vereinigt und mit Hilfe eines Extension Tubes (QIAGEN) unter Vakuum auf eine QIAamp Mini Column (QIAGEN) gegeben. Gewaschen wurde nacheinander mit 1000 $\mu$l (bei Bindung an MagAtrract Partikel) bzw. 750 $\mu$l (bei Bindung an QIAamp Mini Säulen) AW1 (QIAGEN, kommerziell erhältlich), AW2 (QIAGEN, kommerziell erhältlich) und Ethanol. Zum Trocknen wurden die Säulen 3 min bei 14.000 rpm zentrifugiert und die Säulen für 5 min in einen Heizblock bei 56 °C gestellt. Die MagAttract Partikel wurden wie in Beispiel 2 beschrieben behandelt). Eluiert wurde auch hier mit 200 $\mu$l RNase freiem Wasser (Zentrifugation für 1 min bei 14.000 rpm). Die nachfolgenden Real-time PCR ergab dabei das in Fig. 4 gezeigte Bild.

Dieser Versuch zeigt überraschenderweise, dass es bei gleicher Pufferzusammensetzung kaum einen Unterschied macht, ob als feste Phase magnetische Partikel oder eine Silikamembran eingesetzt werden. Bei Zugabe von 1.2 ml Puffer B6 werden längere DNA-Fragmente (ca. 1000 bp) In der erhaltenen DNA-Probe angereichert, während bei Zugabe von 2,0 ml Puffer B6 überraschenderweise umgekehrt kurze DNA-Fragmente (ca. 200 bp) angereichert werden. Bei Einsatz einer Silikamenbran (QIAamp Mini) ist die Ausbeute an DNA insgesamt höher als bei MagAttract Partikeln, allerdings ist auch die größenabhängige DNA-Bindung etwas weniger ausgeprägt. Eine Kombination von magnetischen Partikeln (erste Matrix) und Silikamembran (zweite Matrix) in einer zweistufigen DNA-Extraktion (bei der der DNA-haltige Überstand der 1. Bindung weiterverwendet und an eine Silikamembran gebunden wird) eignet sich folglich hervorragend, um kurze DNA-Fragmente in zwei Schritten effizient anzureichern und damit auch um fötale DNA relativ zu maternaler DNA auf der zweiten Matrix effektiv anzureichern.

### Beispiel 4

Vorgegangen wurde ähnlich wie in Beispiel 3 angegeben, nur dass diesmal mit einer realen Blutprobe gearbeitet wurde und ein zweistufiges Bindeverfahren eingesetzt wurde, Dabei wurde für den 1. Bindeschritt der Probe 1.2 ml Puffer B6 zugegeben, der Durchbruch bzw.

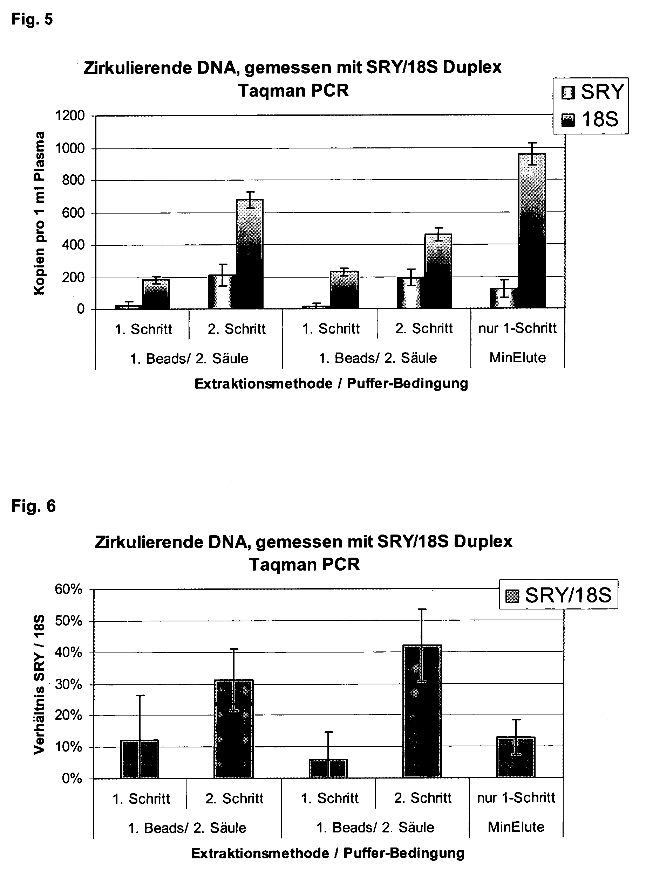

Überstand des 1. Bindeschrittes wurde dann mit zusätzlichem Puffer B6 auf insgesamt 2,0 ml Puffer B6 eingestellt. Ausgangsmaterial war ein Pool aus Plasmaproben schwangerer Frauen des 1. und 2. Trimesters, von denen sicher war, dass sie einen Jungen austrugen. Zum Nachweis der fötalen DNA wurde In der nachfolgenden Real-time PCR der SRY-Locus amplifiziert, zum Nachweis der Gesamt-DNA der 18S Locus (siehe auch Beispiel 1). Zum Vergleich wurde ein 1-Säulen Protokoll nach dem QIAamp MinElute Virus Vacuum Protokoll durchgeführt, welches dem Stand der Technik entspricht. Die Ergebnisse sind in Fig. 5 gezeigt.

Das Ergebnis dieses Experimentes deckt sich mit dem Ergebnis von Beispiel 3, was demonstriert, dass das artifizielle System mit 219 bp und 1018 bp Fragment eine gute Simulation für die reale Situation (Beispiel 4) ist. Während durch die erste Matrix so gut wie keine fötale DNA verloren geht, binden signifikante Mengen der maternalen DNA bereits an die erste Matrix (hier: MagAttract magnetische Partikel). Maternale DNA wird daher effektiv in diesem Schritt (x) abgereichert und ist also während der Aufreinlgung über die zweite Säule bereits depletiert. Im Vergleich zum Stand der Technik (MinElute 1-Schritt) bringt die Zwei-Matrix-Methode nicht nur eine erhöhte absolute Zahl an fötaler DNA, sondern bewirkt durch die zumindest teilweise Depletion der maternalen DNA durch die 1. Matrix auch ein deutlich besseres Verhältnis von fötaler zu maternaler DNA im Eluat, was die Nachweisbarkeit fötaler Erbsubstanz aus maternalem Blut deutlich voranbringt. Fig. 6 verdeutlicht noch einmal das verbesserte Verhältnis von fötaler zu maternaler DNA. Während sich bei der 1-Schritt Aufreinigung gemäß dem Stand der Technik ein Anteil von etwa 15% fötaler DNA im Eluat ergibt (quantifiziert über die SRY/18S duplex Real-time PCR), ist der Anteil bei einer 2-Schritt Aufreinigung mehr als verdoppelt mit einem Anteil von 30 bis 40%.

### Beispiel 5

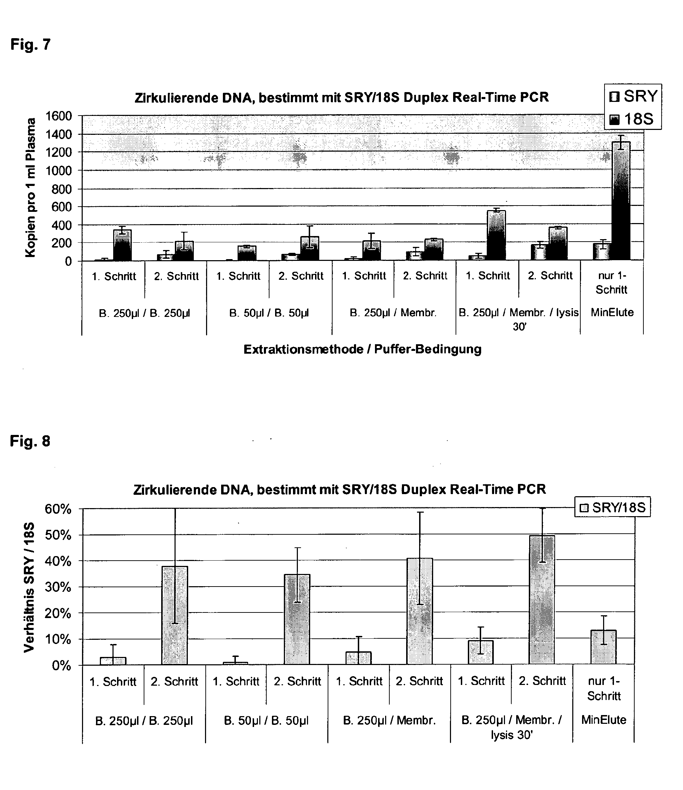

Vorgegangen wurde wie in Beispiel 4 beschrieben, nur dass diesmal ein höheres Volumen an Blutplasma verwendet wurde (3 ml pro Ansatz). Darüber hinaus wurden verschiedene Kombinationen von Bindungsoberflächen (MagAttract Partikel und QIAamp Mini Säulen) sowie verschiedene Mengen an MagAttract miteinander verglichen. Zusätzlich wurde beim Membran-2-Schritt-Protokoll für 30 min lysiert, nicht nur für 15 min. Die Ergebnisse sind in Fig. 7 gezeigt. Dieses Experiment bestätigt die Ergebnisse der vorangegangenen Experimente. Verglichen mit dem 1-Schritt-Protokoll des Standes der Technik ergeben sich mit dem 2-Schritt-Protokoll gemäß der Erfindung immer verbesserte Verhältnisse von fötaler DNA zu maternaler DNA und zwar unabhängig davon, ob im zweiten Bindeschritt magnetische Partikel mit Silikaoberfläche oder Silikamembrane eingesetzt werden. Eine verlängerte Lyse von 30 min scheint dabei noch zusätzlich das Verhältnis von fötaler DNA zu maternaler DNA zugunsten von fötaler DNA zu verbessern, so dass dadurch die maternale DNA deutlich depletiert werden kann. Diese Verbesserung des Verhältnisses ist auch in Fig. 8 gezeigt.

Während beim 1-Matrix Protokoll gemäß dem Stand der Technik sich nur ein Verhältnis von fötaler zu maternaler DNA von etwa 15% ergibt, lässt sich dieses Verhältnis durch das 2-Matrix-Protokoll auf bis zu 50% fötale DNA anheben. Der Anteil der fötalen DNA in der aufgereinigten zirkulierenden DNA aus maternalem Plasma kann also im Vergleich zum Stand der Technik deutlich gesteigert werden.

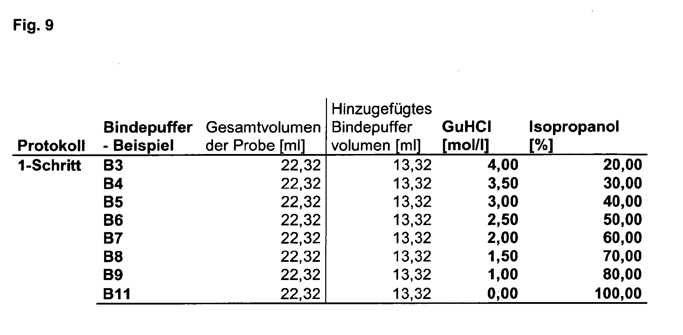

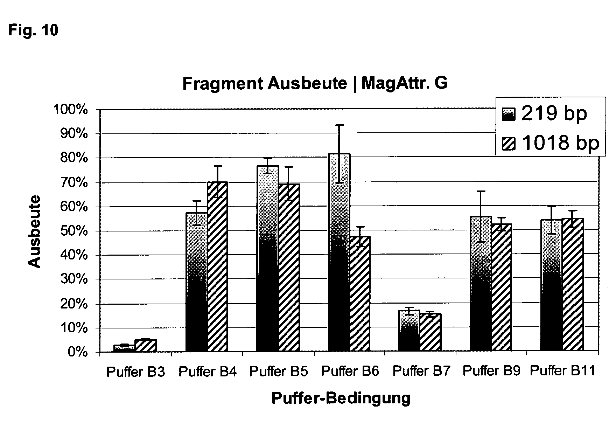

Die in Fig. 9 gezeigte Tabelle zeigt ferner verschiedene Reaktionsbedingungen in der Probe unter denen die Bindung der Nukleinsäuren an das Trägermaterial erfolgt (gemäß dem erfindungsgemäßen 1 - Schrittverfahren). Eingesetzt wurden MagAttract Partikel als Trägermaterial. Die Ergebnisse sind in Fig. 10 gezeigt.

### Beispiel 6

Um die Effizienz des erfindungsgemäßen Verfahrens zur Extraktion von frei zirkulierender DNA aus humanem Plasma zu vergleichen, wurden die folgenden Protokolle miteinander verglichen:

1. Das im Stand der Technik eingesetzte 1-Schritt Verfahren von frei zirkulierender DNA, nämlich das nachfolgend angegebene modifizierte QIAamp MinElute Virus Protokoll

2. Das 1-Schritt Protokoll wie hier beschrieben zur Extraktion/Isolierung frei zirkulierender DNA ("one step").

Für diesen Versuch wurde gepooltes Plasma von männlichen Spendern in vier Extraktionswiederholungen pro Protokoll eingesetzt und getestet. Die DNA Extraktion erfolgte gemäß dem jeweiligen Protokoll. 5 ml Plasma wurde eingesetzt; die DNA wurde In 50 μl eluiert.

**1. QIAamp MInElute Virus Vakuum Protokoll, modifiziert - Stand der Technik**

Die frei zirkulierenden Nukleinsäuren wurden aus 5 ml EDTA Plasma Isoliert. Das Protokoll erfolgte wie folgt:

*Release - Bedingungen*

750 μl QIAGEN Protease (aufgelöst In Protease Solvent) wurde In ein 50 ml Gefäß pipettiert. Danach wurden 5 ml Plasma und 5 ml des Guanidin enthaltenden Puffers AL (mit 5,6 μg carrier RNA) zugegeben. Das Gefäß wurde verschlossen und gut gevortext, um eine homogene Lösung zu erhalten. Die homogene Lösung wurde dann 30 Minuten bei 56°C im Wasserbad inkubiert.

*Zugabe der Markerfragmente*

Danach wurden zum homogenen Gemisch 20 μl einer Markerfragmentmischung hinzugegeben, um die Situation der fötalen Nukleinsäure gemischt mit maternalen Nukleinsäuren zu simulieren. Dazu wurden je 200.000 Kopien von 219 bp (entspricht der fötalen DNA) und 1.018 bp (entspricht der maternalen DNA) Fragmenten hinzugegeben.

*Bindung*

6 ml Ethanol wurde zu dem Lysat hinzugegeben. Das Gemisch wurde gevortext und 5 Minuten auf Eis inkubiert. Das Lysat wurde auf eine QIAamp Minisäule geladen, wobei ein Extension-Tube auf einer QIAvac 24 Vakuumvorrichtung befestigt wurde. Das Lysat wurde durch Anlegung des Vakuums durch die Säule gezogen. Die Extension Tubes wurden vorsichtig entfernt.

*Waschschritte*

600 μl des Puffers AW1 (QIAGEN, kommerziell erhältlich) wurde auf die Säule gegeben und Vakuum angelegt. Dieser Waschschritt wurde mit 750 μl des Puffers AW2 (QIAGEN, kommerziell erhältlich) und mit 750 μl Ethanol wiederholt.
Die Säulen wurden in 2 ml Collection-Tubes platziert, 3 Minuten bei 14.000 rpm zentrifugiert. Dann wurden die Säulen in frische Collection Tubes überführt und in einem Heizblock bei 56°C für 10 Minuten getrocknet.

*Elution*

Die getrockneten Säulen wurden In 1,5 ml Gefäße platziert, und 50 μl des Puffers AVE (QIAGEN, kommerziell erhältlich) auf jede Säule gegeben; Inkubation für 3 Minuten und Zentrifugation für eine Minute bei 14.000 rpm.
Die so gewonnenen Nukleinsäuren befinden sich im Eluat.

**2. Isolierung von frei zirkulierender Nukleinsäure gemäß dem 1-Schritt Verfahren wie hier beschrieben**

*Release*

760 μl QIAGEN Protease (aufgelöst in Protease Solvent) wurde in ein 50 ml Gefäß pipettiert. 5 ml Plasma und 5 ml des Guanidin enthaltenden Lyse/Release Puffers AL (QIAGEN, kommerziell erhältlich, ohne carrier RNA) wurden hinzu gegeben.
Das Gefäß wurde verschlossen und gut gevortext, um eine homogene Lösung zu bilden. Die homogene Lösung wurde 30 Minuten bei 56°C im Wasserbad inkubiert.

*Zugabe der Markerfrsgmente*

Auch hier wurden wiederum 20 μl einer Markerfragmentmischung hinzugegeben (je 200.000 Kopien an 200 bp und 1.000 bp Fragmenten, um das Verhältnis von fötaler zu maternaler DNA zu simulieren).

*Bindung*

Danach wurden durch Bindepufferzugabe folgende Bindebedingungen eingestellt: ca. 25 bis 35% Isopropanol und mehr als 2M chaotrope Verbindungen. Entscheidend sind die Reaktionsbedingungen in der Gesamtprobe und nicht im Puffer, da die Reaktionsbedingungen im Gemisch entscheidend für die Bindungseffizienz der kurzkettigen Nukleinsäuren an das Trägermaterial sind.
Die Probe wurde gevortext und 5 Minuten auf Eis inkubiert.
Das mit dem Bindepuffer gemischte Lysat wurde dann auf eine QIAamp Minisäule mit Extension Tube geladen die auf einer QIAvac 24 Vakuumvorrichtung befestigt war. Das Lysat wurde durch die Säule durch Anlegen von Vakuum gezogen. Die Extension-Tubes wurden dann vorsichtig entfernt.

*Waschschritte*

600 μl eines Waschpuffers, wie beispielsweise AW1 (QIAGEN, kommerziell erhältlich), wurde auf die Säule gegeben und mittels Vakuum abgezogen. Weitere Waschschritte mit 760 μl des Puffers AW2 (QIAGEN, kommerziell erhältlich) und mit 750 μl Ethanol können folgen.
Die gewaschenen Säulen wurden in 2 ml Collection-Tubes platziert und 3 Minuten bei 14.000 rpm zentrifugiert. Die Säulen wurden dann in frische Collection Tubes platziert und in einem Heizblock bel 56°C für 10 Minuten getrocknet.

*Elution*

Die Säulen wurden in 1,5 ml Gefäßen platziert und 50 μl des Elutionspuffers AVE (QIAGEN, kommerziell erhältlich) wurde zu den Säulen gegeben, die Inkubation erfolgte für 3 Minuten, danach erfolgte ein Zentrifugationsschritt für eine Minute bei 14.000 rpm. Die überwiegend kurzkettigen Nukleinsäuren befinden sich im Eluat.

3. Ergebnisse

Die Ausbeute an DNA gemäß den einzelnen Protokollen wurde durch eine quantitative, duplexe, Real-Time PCR gemessen, wobei Taqman Proben eingesetzt wurden (4 PCR Wiederholungen pro DNA Extraktion). Die DNA wurde zum einen durch ein Y-chromosomales Target (DYS14) und ein 18S rDNA spezifisches Target bestimmt. Durch beide Methoden wurde letztlich die Konzentration der DNA in der Probe bestimmt. Da die 18S rDNA auf beiden Chromosomen vorhanden ist, liegt sie in doppelter Menge als das Y-chromosomale Target vor. Die DNA Ausbeute wurde als haploide Genom-Kopien pro ml Plasma angegeben.

Die Anreicherung von kleinen DNA Fragmenten wurde unabhängig bestimmt, in den angereicherten 200 bp und 1.000 bp DNA Fragmenten in einer duplexen, Real-Time PCR.

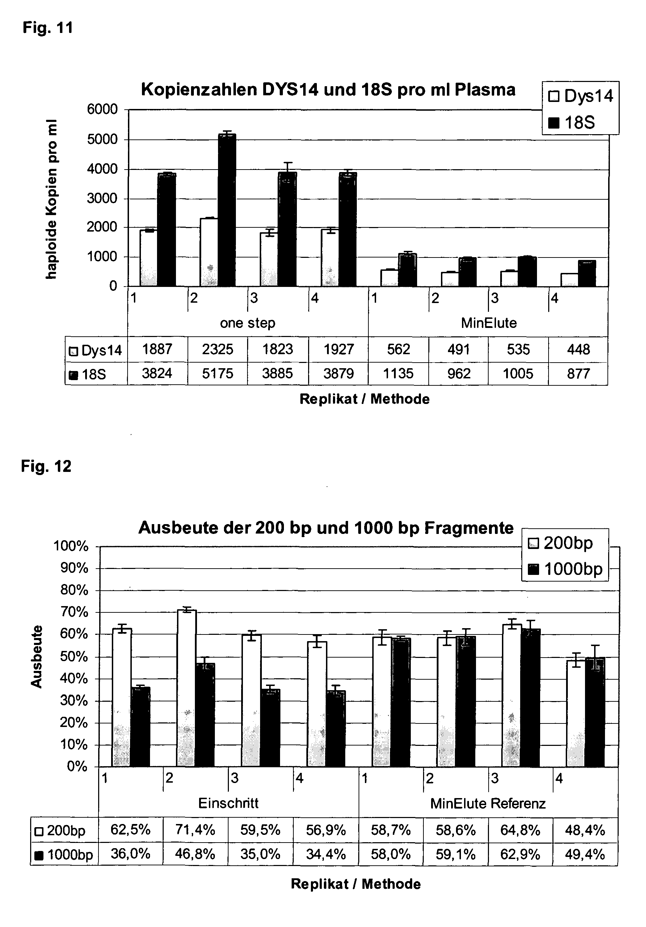

Die Ergebnisse sind In den Figuren 11 ff, dargestellt.

Figur 11 zeigt die DNA Ausbeute pro Probe (4 Proben wurden pro Extraktionsmethode getestet). Gezeigt ist daher der Gesamt-DNA Gehalt, der mittels der einzelnen Methoden erhalten wurde. Wie die Übersicht zeigt, wird mit dem 1-Schritt Verfahren wie hier beschrieben eine deutlich höhere Ausbeute als mit dem im Stand der Technik bekannten 1-Schritt Verfahren (MinElute) erreicht. Die DNA Ausbeute liegt um ein Vielfaches höher, was grade zur Bestimmung von gering exprimierten/vorhandenen Nukleinsäuren von besonderem Vorteil ist.

Figur 12 zeigt die Ausbeute der eingespeisten 200 bp (simuliert die fötalen Nukleinsäuren) und 1.000 bp (simuliert die langkettigen maternalen Fragmente) DNA Fragmente. Wie oben dargelegt, wurden die 200 bp und die 1.000 bp Fragmente in einem Verhältnis von 1:1 eingespeist, nämlich je 200.000 Kopien. Die in Figur 12 gezeigte Grafik zeigt, ob bei den aufgereinigten Nukleinsäuren das Verhältnis von kurzkettigen zu langkettigen Nukleinsäuren nach wie vor 1:1 ist, oder ob eine Anreicherung der kurzkettigen Nukleinsäuren erzielt wurde. Wie Figur 12 zeigt, hat das 1-Schritt Verfahren des Standes der Technik (MinElute) ein Verhältnis von langkettigen zu kurzkettigen Nukleinsäuren von ca. 1:1. Es wird daher keine Anreicherung der kurzkettigen Nukleinsäuren erzielt. Unter der Fig. 12 ist auch noch tabellarlsch gezeigt, wie viel der einzelnen Fragmente (200 bp und 1.000 bp) aus der Probe gewonnen wurde. So gibt beispielsweise die Angabe, dass 62,5% an 200 bp Fragmenten und 36% von 1.000 bp Fragmenten aufgereinigt wurden an, dass 62,5% der anfänglich eingespeisten 200.000 Kopien der 200 bp Fragmente aufgereinigt und damit isoliert wurden und 36% der 200.000 Kopien der 1.000 bp Fragmente. Wie gezeigt, wird mit dem 1-Schritt Verfahren wie hier beschrieben eine deutliche Anreicherung der kurzkettigen Nukleinsäuren gegenüber der langkettigen Nukleinsäuren erzielt. Das Verhältnis ist nicht länger 1:1 (wie eingespeist), sondern es wurden deutlich mehr kurzkettige Nukleinsäuren aufgereinigt und damit im Eluat angereichert. Das Verfahren ist daher dem Stand der Technik deutlich überlegen.

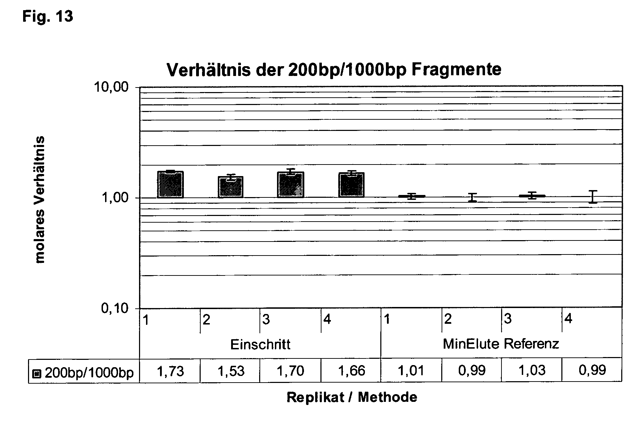

Das Verhältnis der aufgereinigten 200 bp zu 1.000 bp Fragmente ist zusätzlich in Figur 13 gezeigt. Während beim Im Stand der Technik bekannten 1-Schritt Verfahren Werte von ungefähr 1 erzielt werden, ist das Verhältnis bel dem 1-Schritt Verfahren wie hier beschrieben deutlich in Richtung der kurzkettigen Nukleinsäuren verschoben, die entsprechend bevorzugt angereichert/isoliert werden. Bei dem erfindungsgemäßen 2-Schritt Verfahren, bei dem die langkettigen Nukleinsäuren In einem Vorschritt zunächst abgereichert werden, wird sogar eine 5 bis 10-fache Anreicherung erzielt.

Wie die Ergebnisse zeigen, ist das hier beschriebene 1-Schritt Verfahren dem im Stand der Technik bekannten 1-Schritt Verfahren deutlich überlegen. Das hier beschriebene 1-Schritt Verfahren reichert kleine Nukleinsäuren während der Präparation an, was auf die einzigartigen Reaktionsbedingungen während der Bindung zurückzuführen ist, die zu einer bevorzugten Bindung von kurzkettigen Nukleinsäuren führt.

**Beispiel 7**

Nachfolgend ist die Isolation von Nukleinsäuren, insbesondere zirkulierender RNA aus 5 ml Plasma oder Serum beschrieben.

1350 μl Puffer AVE (QIAGEN, kommerziell erhältlich, enthält Guanidin) wird In ein Gefäß mit 1350 μg lyophilisierter Carrier RNA gegeben, so dass eine Lösung von 1 μg/ml erhalten wird. Die Lösung von 1 μg/μl wird dann mit dem Puffer AL (QIAGEN, kommerziell erhältlich, enthält Guanidin) vermischt. Das Mischungsverhältnis wird je nach Anzahl der Proben angepasst. Für die Behandlung einer Probe werden 8,5 ml des Puffers AL mit 5,6 μl des Puffers AVE gemischt. Für mehr Proben müssen die Verhältnisse entsprechend angepasst werden. Das Gefäß wird zur Vermischung 10 Mal hin und her geschwenkt.

6 ml Protease Lösung (QIAGEN, kommerziell erhältlich) wird zu einer lyophilisierten QIAGEN Protease (7.5 A.U, kommerziell erhältlich) gegeben und vorsichtig gemischt.

500 μl QIAGEN Protease werden in ein 50 ml Gefäß (Tube) pipettiert und die 5 ml Plasma hinzu gegeben. Danach werden 8,5 ml des Puffers AL, gemischt mit Carrier RNA (siehe oben) hinzu gegeben und gevortext, um die Substanzen zu mischen.

Die gemischte Probe wird für 30 Minuten bei 56°C inkubiert.

7,5 ml eines Bindepuffers wird zu dem Lysat hinzu gegeben (enthält ca. 0,5 bis 1,5 mol/l Guanidinum, vorzugsweise um 1 mol/l und Isopropanol ca. 60 - 90 % (v/v); vorzugsweise mehr als 70%). Die Mischung wird für 30 Sekunden gevortext und 5 Minuten auf Eis inkubiert. Eine QIAamp Minisäule wird in einen VacConnector auf einem QIAvac 24 Plus inseriert und ein Extension Tube in der offenen QIAamp Minisäule platziert.

Das Lysat wird in das Extension Tube gefüllt und Vakuum angelegt. Sobald das Lysat durch die Säulen durchgezogen wurde, wird die Vakuumpumpe ausgestellt und der Druck ausgeglichen. Das Extension Tube wird verworfen.

Die Säule wird aus dem Vakuumträger entfernt und in ein 2 ml Auffanggefäß transferiert. Die Säule wird 1min bei 14000 rpm zentrifugiert.

Zur Präparation von RNA wird eine 10 $\mu$l DNAse I Stock Lösung zu 70 $\mu$l des Puffers RDD (QIAGEN, kommerziell erhältlich) pipettiert. Gemischt wird durch Schwenken des Tubes. Der Puffer RDD wird mit dem RNAse-free DNAse Set (QIAGEN, CAT.No.79254) geliefert.

Die Säulen werden wieder auf dem QIAvac 24 Plus Vakuumträger platziert. Die DNAse I Mischung wird direkt auf die QIAamp Mini Silikagelmembran pipettiert und bei gemäßigten Temperaturen (20 bis 30°C) für 15 Minuten inkubiert.

Danach werden 600$\mu$l des Puffers AW1 (QIAGEN, kommerziell erhältlich) auf die QIAamp Minisäule pipettiert. Danach wird Vakuum appliziert, um die Mischung durch die Säule zu ziehen. Im Anschluss werden 750 $\mu$l des Puffers AW2 (QIAGEN, kommerziell erhältlich) hinzu gegeben und durch Anlegen von Vakuum durch die Säule gezogen.

Im Anschluss werden 750 $\mu$l Ethanol (96-100%) auf die Säule gegeben und mittels Vakuum durchgezogen. Die QIAamp Minisäule wird im Anschluss aus dem Vakuumträger entfernt und der VacConnector wird verworfen. Die Säulen werden in ein sauberes 2 ml Auffanggefäß platziert und bei 20.000x g, 14.000 rpm für 3 Minuten zentrifugiert.

Die Säule wird in ein neues 2 ml Auffanggefäß platziert und bel geöffnetem Deckel für 10 Minuten bei 56°C getrocknet. Die QIAamp Minisäule wird dann in ein sauberes 1,5 ml Mikrozentrifugationsgefäß platziert und das Sammelgefäß wird verworfen. 20 bis 60 $\mu$l des Puffers AVE (QIAGEN, kommerziell erhältlich) wird auf die Mitte der QIAamp Minimembran pipettiert. Bei geschlossenem Deckel erfolgt eine Inkubation für 3min.

Im Anschluss erfolgt ein Zentrifugationsschritt bei 20.000 x g, 14.000 rpm für 1min um die RNA zu aluieren. Im Anschluss wird ein RNAse Inhibitor dazugegeben.

Mit dem entsprechenden Protokoll lässt sich hervorragend kurzkettige RNA aufreinigen.

**Patentansprüche**

1. Verfahren zur Anreicherung von kurzkettigen Nukleinsäuren, insbesondere DNA und/oder RNA, mit einer Länge von < 1000bp, aus einem nukleinsäurehaltigen Ausgangsmaterial mit folgenden Verfahrensschritten:

   (x) Bindung von langkettigen Nukleinsäuren an ein nukleinsäurebindendes Trägermaterial, indem man das Ausgangsmaterial in Gegenwart wenigstens einer chaotropen Verbindung und wenigstens eines verzweigten und/oder unverzweigten Alkohols mit dem nukleinsäurebindenden Trägermaterial in Kontakt bringt, wobei der Alkohol in einer Konzentration ≤ 25% (v/v) vorliegt;
   (a) Bindung der im Durchbruch/Überstande aus Schritt (x) enthaltenden kurzkettigen Nukleinsäuren an ein nukleinsäurebindendes Trägermaterial, indem man den Durchbruch/Überstand in Gegenwart wenigstens einer chaotropen Verbindung und wenigstens eines verzweigten und/oder unverzweigten Alkohols mit dem nukleinsäurebindenden Trägermaterial in Kontakt bringt, wobei der Alkohol in einer Konzentration von 25 bis 40% (v/v) vorliegt;
   (b) optional Elution der gebundenen kurzkettigen Nukleinsäuren von dem nukleinsäurebindenden Trägermaterial.

2. Verfahren nach Anspruch 1, **dadurch gekennzeichnet, dass** in Schritt (x) der Alkohol in der Mischung in einer Konzentration von ≤ 20% (v/v) vorliegt.

3. Verfahren nach Anspruch 1 oder 2, **dadurch gekennzeichnet, dass** die Alkoholkonzentration während der Bindung der Nukleinsäuren an das Trägermaterial in Schritt (x) niedriger ist als die Alkoholkonzentration während der Bindung der Nukleinsäuren an das Trägermaterial in Schritt (a).

4. Verfahren nach einem oder mehreren der Ansprüche 1 bis 3, **gekennzeichnet durch** eines oder mehrere der folgenden Merkmale:

   a. dass die Konzentration der chaotropen Verbindungen in der Mischung in Schritt (x) und/oder Schritt (a) ≥ 2 mol/l, ≥ 2,4 mol/l oder ≥ 2,6 mol/l ist; und/oder
   b. dass die Konzentration der chaotropen Verbindungen in der Mischung in Schritt (x) und/oder Schritt (a) ≤4 mol/l, ≤3,5 mol/l, ≤ 3,2 mol/l oder ≤3,1 mol/l ist; und/oder

c. dass die Konzentration der chaotropen Verbindungen in Schritt (a) ≥ der Konzentration der chaotropen Verbindungen in Schritt (x) ist; und/oder

d. dass wenigstens 30%, 50% und vorzugsweise wenigstens 60% der in dem Ausgangsmaterial vorhandenen kurzkettigen Nukleinsäuren mittels des Verfahrens isolierbar sind; und/oder

e. dass kurzkettige Nukleinsäuren mit einer Länge isoliert und/oder angereichert werden, die ausgewählt sind aus der Gruppe von Nukleinsäuren mit einer Länge von ≤ 500bp, ≤ 400bp und/oder ≤ 300bp und/oder ≥ 50bp und/oder ≥ 100bp.

5. Verfahren nach einem oder mehreren der Ansprüche 1 bis 4, **dadurch gekennzeichnet, dass** die chaotrope Verbindung ausgewählt ist aus Natriumiodid, Natriumperchlorat, Guanidiniumthiocyanat, Guanidiniumisothiocyanat und Guanidiniumhydrochlorid.

6. Verfahren nach einem oder mehreren der Ansprüche 1 bis 5, **dadurch gekennzeichnet, dass** die Konzentration der chaotropen Verbindung in Schritt a) bei ≥ 2,5mol/l und ≤ 3,1mol/l liegt und die chaotrope Verbindung vorzugsweise Guanidinumhydrochlorid ist.

7. Verfahren nach einem oder mehreren der Ansprüche 1 bis 6, **dadurch gekennzeichnet, dass** es sich bei dem Alkohol um Isopropanol handelt.

8. Verfahren nach einem oder mehreren der Ansprüche 1 bis 7, **dadurch gekennzeichnet, dass** in Schritt (a) der Alkohol in der Probenmischung in einer Konzentration vorliegt, die ausgewählt ist aus der Gruppe ≥ 25% bis ≤ 35% (v/v), ≥ 25% bis ≤ 32% (v/v) und ≥ 28% bis ≤ 32% (v/v).

9. Verfahren nach einem oder mehreren der Ansprüche 1 bis 8, **dadurch gekennzeichnet, dass** keine Phenol-Extraktion durchgeführt wird.

10. Verfahren nach einem oder mehreren der Ansprüche 1 bis 9, **dadurch gekennzeichnet, dass** die Nukleinsäuren extrazelluläre Nukleinsäuren sind.

11. Verfahren nach einem oder mehreren der Ansprüche 1 bis 10, **dadurch gekennzeichnet, dass** die zu isolierende/aufzureinigende Nukleinsäure DNA ist.

12. Verfahren nach einem oder mehreren der Ansprüche 1 bis 11, **dadurch gekennzeichnet, dass** die kurzkettigen Nukleinsäuren einzelsträngig oder doppelsträngig sind.

13. Verfahren nach einem oder mehreren der Ansprüche 1 bis 12, **dadurch gekennzeichnet, dass** das nukleinsäurebindende Trägermaterial ausgewählt ist aus der Gruppe von silikahaltigen Materialien, Silikagel, Glas, Zeolith, Aluminiumoxid, Titandioxid, Zirkoniumdioxid, Kaolin, Kieselgel, magnetischen Partikeln, insbesondere magnetischen Silika- oder Glaspartikeln, Keramik oder polymeren Trägermaterialien.

14. Verfahren nach einem oder mehreren der Ansprüche 1 bis 13, **dadurch gekennzeichnet, dass** während der Bindung Detergenzien, vorzugsweise nichtionische Detergenzien, anwesend sind.

15. Verfahren nach einem oder mehreren der Ansprüche 1 bis 14 zur Isolierung von frei zirkulierenden Nukleinsäuren, vorzugsweise aus Plasma und/oder Serum.

16. Verfahren nach einem oder mehreren der Ansprüche 1 bis 15, zur Isolierung von fötaler DNA aus maternalen Blutproben, insbesondere Blutplasma.

17. Verfahren nach einem oder mehreren der Ansprüche 1 bis 16 zur Isolierung von DNA, wobei in Schritt (x) magnetische Partikel als Trägermaterial eingesetzt werden und in Schritt (a) eine Silikamembran.

18. Verfahren nach einem oder mehreren der Ansprüche 1 bis 17, **dadurch gekennzeichnet, dass** die in Schritt (x) gebundenen langkettigen Nukleinsäuren vom Trägermaterial eluiert werden.

19. Verfahren zur Anreicherung von fötalen Nukleinsäuren aus einer Blutprobe, insbesondere aus Blutplasma oder -serum, **dadurch gekennzeichnet, dass** ein Verfahren gemäß einem oder mehreren der Ansprüche 1 bis 18 durchgeführt wird.

20. Verfahren nach einem oder mehreren der Ansprüche 1 bis 19 zur Anwendung in der Diagnostik.

**Claims**

1. Method for enriching short-chain nucleic acids, in particular DNA and/or RNA, with a length of < 1000bp from a nucleic acid-containing starting material comprising the following method steps:

(x) Binding of long-chain nucleic acids to a nucleic acid-binding support material, by contracting the starting material in the presence of at least one chaotropic compound and at least one branched and/or unbranched alcohol with the nucleic acid-binding support material, wherein the alcohol is present in a concentration of ≤ 25% (v/v);

(a) Binding of short-chain nucleic acids contained in the breakthrough/supernatant from step (x) to a nucleic acid-binding support material, by contacting the breakthrough/supernatant in the presence of at least one chaotropic compound and at least one branched and/or unbranched alcohol with the nucleic acid-binding support material, wherein the alcohol is present in a concentration of 25 to 40% (v/v);

(b) Optionally eluting the bound short-chain nucleic acids from the nucleic acid-binding support material.

2. Method according to claim 1, **characterised in that** in step (x) the alcohol in the mixture is present in a concentration of ≤ 20% (v/v).

3. Method according to claim 1 or 2, **characterised in that** the alcohol concentration during the binding of the nucleic acids to the support material in step (x) is lower than the alcohol concentration during the binding of the nucleic acids to the support material in step (a).

4. Method according to one or more of claims 1 to 3, **characterised by** one or more of the following features:

   a. that the concentration of the chaotropic compounds in the mixture in step (x) and/or step (a) is ≥ 2 mol/l, ≥ 2.4 mol/l or ≥ 2.6 mol/l; and/or
   b. that the concentration of the chaotropic compounds in the mixture in step (x) and/or step (a) is ≤ 4 mol/l, ≤ 3.5 mol/l, ≤ 3.2 mol/l or ≤ 3.1 mol/l; and/or
   c. that the concentration of the chaotropic compounds in step (a) is ≥ the concentration the chaotropic compounds in step (x); and/or
   d. that at least 30%, 50% and preferably at least 60% of the short-chain nucleic acids present in the starting material can be isolated by the method; and/or
   e. that short-chain nucleic acids are isolated and/or enriched that are selected from the group of nucleic acids with a length of ≤ 500bp, ≤ 400bp and/or ≤ 300bp and/or ≥ 50bp and/or ≥ 100bp.

5. Method according to one or more of claims 1 to 4, **characterised in that** the chaotropic compound is selected from sodium iodide, sodium perchlorate, guanidinium thiocyanate, guanidinium isothiocyanate and guanidinium hydrochloride.

6. Method according to one or more of claims 1 to 5, **characterised in that** the concentration of the chaotropic compound in step a) is ≥ 2.5mol/l and ≤ 3.1 mol/l and the chaotropic compound is preferably guanidinum hydrochloride.

7. Method according to one or more of claims 1 to 6, **characterised in that** the alcohol used is isopropanol.

8. Method according to one or more of claims 1 to 7, **characterised in that** in step (a) the alcohol in the sample mixture is present in a concentration selected from the group ≥ 25% to ≤ 35% (v/v), ≥ 25% to ≤ 32% (v/v) and ≥ 28% to ≤ 32% (v/v).

9. Method according to one or more of claims 1 to 8, **characterised in that** no phenol extraction is performed.

10. Method according to one or more of claims 1 to 9, **characterised in that** the nucleic acids are extracellular nucleic acids.

11. Method according to one or more of claims 1 to 10, **characterised in that** the nucleic acid to be isolated/purified is DNA.

12. Method according to one or more of claims 1 to 11, **characterised in that** the short-chain nucleic acids are single-stranded or double-stranded.

13. Method according to one or more of claims 1 to 12, **characterised in that** the nucleic acid-binding support material is selected from the group of silica-containing materials silica gel, glass, zeolite, aluminium oxide, titanium dioxide, zirconium dioxide, kaolin, silica gel, magnetic particles, in particular magnetic silica or glass particles, ceramics or polymeric support materials.

14. Method according to one or more of claims 1 to 13, **characterised in that** during binding, detergents, preferably non-ionic detergents, are present.

15. Method according to one or more of claims 1 to 14 for isolating free circulating nucleic acids, preferably from plasma and/or serum.

16. Method according to one or more of claims 1 to 15, for isolating foetal DNA from maternal blood samples, in particular from blood plasma.

17. Method according to one or more of claims 1 to 16 for isolating DNA, wherein in step (x) magnetic particles are used as support material and in step (a) a silica membrane.

18. Method according to one or more of claims 1 to 17, **characterised in that** the long-chain nucleic acids bound in step (x) are eluted from the support material.

**19.** Method for enriching foetal nucleic acids out of a blood sample, in particular from blood plasma or -serum, **characterised in that** a method according to one or more of claims 1 to 18 is performed.

**20.** Method according to one or more of claims 1 to 19 for use in diagnostics.

**Revendications**

**1.** Procédé pour l'enrichissement d'acides nucléiques à courte chaîne, en particulier d'ADN et/ou d'ARN, présentant une longueur < 1000 pb, à partir d'un matériau de départ contenant des acides nucléiques, présentant les étapes de procédé suivantes consistant à :

> (x) lier des acides nucléiques à longue chaîne à un matériau support liant les acides nucléiques, en ce qu'on met en contact le matériau de départ en présence d'au moins un composé chaotropique et d'au moins un alcool ramifié et/ou non ramifié avec le matériau support liant les acides nucléiques, l'alcool se trouvant en une concentration $\leq$ 25% (v/v) ;
> (a) lier des acides nucléiques à courte chaîne contenus dans la fraction passée/surnageante de l'étape (x) à un matériau support liant les acides nucléiques, en ce qu'on met en contact la fraction passée/surnageante en présence d'au moins un composé chaotropique et d'au moins un alcool ramifié et/ou non ramifié avec le matériau support liant les acides nucléiques, l'alcool se trouvant en une concentration de 25 à 40% (v/v) ;
> (b) éluer éventuellement les acides nucléiques à courte chaîne liés au matériau support liant les acides nucléiques.

**2.** Procédé selon la revendication 1, **caractérisé en ce que**, dans l'étape (x), l'alcool dans le mélange se trouve en une concentration $\leq$ 20% (v/v).

**3.** Procédé selon la revendication 1 ou 2, **caractérisé en ce que** la concentration en alcool pendant la liaison des acides nucléiques au matériau support dans l'étape (x) est inférieure à la concentration en alcool pendant la liaison des acides nucléiques au matériau support dans l'étape (a).

**4.** Procédé selon l'une ou plusieurs des revendications 1 à 3, **caractérisé par** une ou plusieurs des caractéristiques suivantes :

> a. la concentration en composés chaotropiques dans le mélange dans l'étape (x) et/ou dans l'étape (a) est $\geq$ 2 moles/l, $\geq$ 2,4 moles/l ou $\geq$ 2,6 moles/l ; et/ou
> b. la concentration en composés chaotropiques dans le mélange dans l'étape (x) et/ou dans l'étape (a) est $\leq$ 4 moles/l, $\leq$ 3,5 moles/l, $\leq$ 3,2 moles/l ou $\leq$ 3,1 moles/l ; et/ou
> c. la concentration en composés chaotropiques dans l'étape a) est $\geq$ à la concentration en composés chaotropiques dans l'étape (x) ; et/ou
> d. au moins 30%, 50% et de préférence au moins 60% des acides nucléiques à courte chaîne présents dans le matériau de départ peuvent être isolés au moyen du procédé ; et/ou
> e. les acides nucléiques à courte chaîne qui sont isolés et/ou enrichis sont ceux qui sont choisis dans le groupe des acides nucléiques d'une longueur $\leq$ 500 pb, $\leq$ 400 pb et/ou $\leq$ 300 pb et/ou $\geq$ 50 pb et/ou $\geq$ 100 pb.

**5.** Procédé selon l'une ou plusieurs des revendications 1 à 4, **caractérisé en ce que** le composé chaotropique est choisi parmi l'iodure de sodium, le perchlorate de sodium, le thiocyanate de guanidinium, l'isothiocyanate de guanidinium et le chlorhydrate de guanidinium.

**6.** Procédé selon l'une ou plusieurs des revendications 1 à 5, **caractérisé en ce que** la concentration en composé chaotropique dans l'étape a) est $\geq$ 2,5 moles/l et $\leq$ 3,1 moles/l et le composé chaotropique est de préférence le chlorhydrate de guanidinium.

**7.** Procédé selon l'une ou plusieurs des revendications 1 à 6, **caractérisé en ce qu'**il s'agit, pour l'alcool, d'isopropanol.

**8.** Procédé selon l'une ou plusieurs des revendications 1 à 7, **caractérisé en ce que** dans l'étape (a), l'alcool dans le mélange d'échantillon se trouve en une concentration qui est choisie dans le groupe $\geq$ 25% à $\leq$ 35% (v/v), $\geq$ 25% à $\leq$ 32% (v/v) et $\geq$ 28% à $\leq$ 32% (v/v).

**9.** Procédé selon l'une ou plusieurs des revendications 1 à 8, **caractérisé en ce qu'**on ne réalise pas d'extraction de phénol.

**10.** Procédé selon l'une ou plusieurs des revendications 1 à 9, **caractérisé en ce que** les acides nucléiques sont des acides nucléiques extracellulaires.

**11.** Procédé selon l'une ou plusieurs des revendications 1 à 10, **caractérisé en ce que** l'acide nucléique à isoler/purifier est de l'ADN.

**12.** Procédé selon l'une ou plusieurs des revendications 1 à 11, **caractérisé en ce que** les acides nucléiques à courte chaîne sont de type monobrin ou de type double brin.

**13.** Procédé selon l'une ou plusieurs des revendications 1 à 12, **caractérisé en ce que** le matériau support liant les acides nucléiques est choisi dans le groupe formé par les matériaux contenant de la silice, le silicagel, le verre, la zéolithe, l'oxyde d'aluminium, le dioxyde de titane, le dioxyde de zirconium, le kaolin, le gel silicique, les particules magnétiques, en particulier les particules magnétiques de silice ou de verre, la céramique ou les matériaux support polymères.

**14.** Procédé selon l'une ou plusieurs des revendications 1 à 13, **caractérisé en ce que** des détergents, de préférence des détergents non ioniques, sont présents pendant la liaison.

**15.** Procédé selon l'une ou plusieurs des revendications 1 à 14 pour l'isolement d'acides nucléiques en circulation libre, de préférence de plasma et/ou de sérum.

**16.** Procédé selon l'une ou plusieurs des revendications 1 à 15 pour l'isolement d'ADN foetal à partir d'échantillons sanguins maternels, en particulier le plasma sanguin.

**17.** Procédé selon l'une ou plusieurs des revendications 1 à 16 pour l'isolement d'ADN, les particules métalliques étant utilisées comme matériau support dans l'étape (x) et une membrane de silice étant utilisée dans l'étape (a).

**18.** Procédé selon l'une ou plusieurs des revendications 1 à 17, **caractérisé en ce que** les acides nucléiques à longue chaîne liés dans l'étape (x) sont élués du matériau support.

**19.** Procédé pour l'enrichissement d'acides nucléiques foetaux d'un échantillon sanguin, en particulier de plasma ou de sérum sanguin, **caractérisé en ce qu'**on réalise un procédé selon l'une ou plusieurs des revendications 1 à 18.

**20.** Procédé selon l'une ou plusieurs des revendications 1 à 19 pour une utilisation en diagnostic.

**Fig. 1**

### fötale DNA pro Fraktion as % der gesamten fötalen DNA

☐ pool A
☒ pool B
■ pool C

DNA Fragmentgröße

**Fig. 2**

### Gesamt-DNA in % pro Fraktion

☐ pool A
☒ pool B
■ pool C

DNA Fragmentgröße

**Fig. 3**

### DNA Fragment-Ausbeute / MagAttract G

Legend: ■ 219 bp  ▨ 1018 bp

Y-axis: Ausbeute (100%, 90%, 80%, 70%, 60%, 50%, 40%, 30%, 20%, 10%, 0%)

X-axis categories: B11 | 1.   B6 | 2.   B11 | 1.   B11 | 2.

X-axis label: Bindepuffer-Bedingung

**Fig. 4**

### DNA Fragmentausbeute / MagAttract / QIAamp Mini

Legend: ☐ 219 bp  ■ 1018 bp

Y-axis: Ausbeute (100%, 90%, 80%, 70%, 60%, 50%, 40%, 30%, 20%, 10%, 0%)

X-axis categories: 2.0 ml B6 | 1.2 ml B6 | 2.0 ml B6 | 1.2 ml B6

Grouping: QIAamp Mini | MagAttract G

X-axis label: Bindebuffer-Bedingung

**Fig. 5**

Zirkulierende DNA, gemessen mit SRY/18S Duplex Taqman PCR

**Fig. 6**

Zirkulierende DNA, gemessen mit SRY/18S Duplex Taqman PCR

**Fig. 7**

Zirkulierende DNA, bestimmt mit SRY/18S Duplex Real-Time PCR

☐ SRY
■ 18S

**Fig. 8**

Zirkulierende DNA, bestimmt mit SRY/18S Duplex Real-Time PCR

☐ SRY/18S

**Fig. 9**

| Protokoll | Bindepuffer - Beispiel | Gesamtvolumen der Probe [ml] | Hinzugefügtes Bindepuffer volumen [ml] | GuHCl [mol/l] | Isopropanol [%] |
|---|---|---|---|---|---|
| 1-Schritt | B3 | 22,32 | 13,32 | 4,00 | 20,00 |
| | B4 | 22,32 | 13,32 | 3,50 | 30,00 |
| | B5 | 22,32 | 13,32 | 3,00 | 40,00 |
| | B6 | 22,32 | 13,32 | 2,50 | 50,00 |
| | B7 | 22,32 | 13,32 | 2,00 | 60,00 |
| | B8 | 22,32 | 13,32 | 1,50 | 70,00 |
| | B9 | 22,32 | 13,32 | 1,00 | 80,00 |
| | B11 | 22,32 | 13,32 | 0,00 | 100,00 |

**Fig. 10**

Fragment Ausbeute | MagAttr. G

**Fig. 11**

## Kopienzahlen DYS14 und 18S pro ml Plasma

Legend: □ Dys14  ■ 18S

Y-axis: haploide Kopien pro ml (6000, 5000, 4000, 3000, 2000, 1000, 0)

|  | 1 | 2 | 3 | 4 | 1 | 2 | 3 | 4 |
|---|---|---|---|---|---|---|---|---|
| | | one step | | | | MinElute | | |
| □ Dys14 | 1887 | 2325 | 1823 | 1927 | 562 | 491 | 535 | 448 |
| ■ 18S | 3824 | 5175 | 3885 | 3879 | 1135 | 962 | 1005 | 877 |

**Replikat / Methode**

**Fig. 12**

## Ausbeute der 200 bp und 1000 bp Fragmente

Legend: ▨ 200bp  ■ 1000bp

Y-axis: Ausbeute (100%, 90%, 80%, 70%, 60%, 50%, 40%, 30%, 20%, 10%, 0%)

|  | 1 | 2 | 3 | 4 | 1 | 2 | 3 | 4 |
|---|---|---|---|---|---|---|---|---|
| | | Einschritt | | | | MinElute Referenz | | |
| □ 200bp | 62,5% | 71,4% | 59,5% | 56,9% | 58,7% | 58,6% | 64,8% | 48,4% |
| ■ 1000bp | 36,0% | 46,8% | 35,0% | 34,4% | 58,0% | 59,1% | 62,9% | 49,4% |

**Replikat / Methode**

**Fig. 13**

**Verhältnis der 200bp/1000bp Fragmente**

| 200bp/1000bp | 1,73 | 1,53 | 1,70 | 1,66 | 1,01 | 0,99 | 1,03 | 0,99 |
|---|---|---|---|---|---|---|---|---|

**Replikat / Methode**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5234809 A, Boom
- WO 9311221 A
- EP 1690938 A
- WO 9961603 A
- US 2007106071 A
- DE 102006045391
- WO 2007065934 A
- WO 0171732 A